(19) Europäisches Patentamt
European Patent Office
Office européen des brevets

(11) Publication number: 0 312 157
A2

(12) EUROPEAN PATENT APPLICATION

(21) Application number: 88202210.6

(51) Int. Cl.4: C07K 5/02 , A61K 37/64

(22) Date of filing: 05.10.88

(30) Priority: 13.10.87 US 107212

(43) Date of publication of application:
19.04.89 Bulletin 89/16

(84) Designated Contracting States:
CH DE FR GB IT LI NL

(71) Applicant: MERCK & CO. INC.
126, East Lincoln Avenue P.O. Box 2000
Rahway New Jersey 07065-0900(US)

(72) Inventor: Chakravarty, Prasun K.
16 Churchill Road
Edison New Jersey 08820(US)
Inventor: Greenlee, William J.
115 Herrick Avenue
Teaneck New Jersey 07666(US)
Inventor: Parsons, William H.
2171 Oliver Street
Rahway New Jersey 07065(US)
Inventor: Schoen, William
196 White Birch Road
Edison New Jersey 08837(US)

(74) Representative: Hesketh, Alan, Dr.
European Patent Department Merck & Co.,
Inc. Terlings Park Eastwick Road
Harlow Essex, CM20 2QR(GB)

(54) **Tetrapeptide renin inhibitors having a novel c-terminal amino acid.**

(57) Tetrapeptide enzyme inhibitors of the formula:

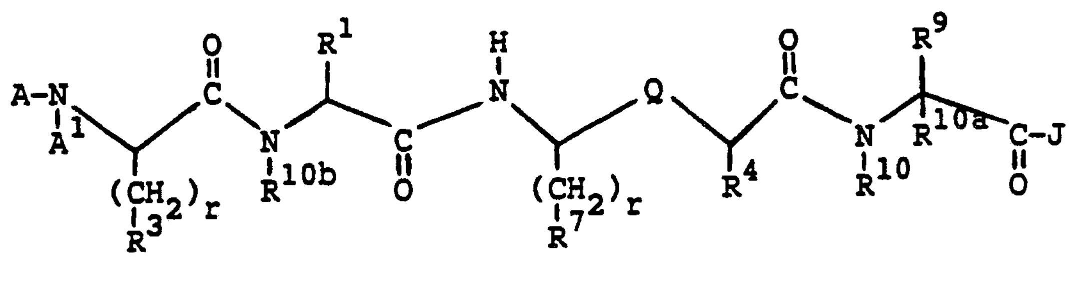

and analogs thereof which inhibit renin and are useful for treating various forms of renin-associated hypertension, hyperaldosteronism and congestive heart failure; compositions containing these renin-inhibitory peptides, optionally with other antihypertensive agents; and methods of treating hypertension, hyperaldosteronism or congestive heart failure or of establishing renin as a causative factor in these problems employing the novel tetrapeptides.

EP 0 312 157 A2

# TETRAPEPTIDE RENIN INHIBITORS HAVING NOVEL C-TERMINAL AMINO ACID AMIDES

The present invention is concerned with novel peptides which inhibit the angiotensinogen-cleaving action of the proteolytic enzyme, renin, with pharmaceutical compositions containing the novel peptides of the present invention as active ingredients, with methods of treating renin-associated hypertension, hyperaldosteronism, and congestive heart failure, with diagnostic methods which utilize the novel peptides of the present invention, and with methods of preparing the novel peptides of the present invention.

## BACKGROUND OF THE INVENTION

Renin is an endopeptidase (molecular weight about 40,000) produced and secreted by the juxtaglomerular cells of the kidney. Renin has a high specificity for and cleaves the naturally-occurring plasma glycoprotein, angiotensinogen, at only the 10, 11 peptide bond, i.e., between the l0th (Leu) and llth (Leu) amino acid residues in the equine substrate, as described by Skeggs et al, J. Exper. Med. 1957, 106, 439, or between Leu 10 and Val 11 in the human renin substrate, as elucidated by Tewksbury et al., Circulation 59, 60, Supp. II: 132, Oct. 1979.

This cleavage of its protein substrate, angiotensinogen, by the aspartic proteinase, renin, splits off the decapeptide, angiotensin I, which itself is thought to be hemodynamically-inactive, but which is converted in the lungs, kidney or other tissue by angiotensin-converting enzyme (ACE) to the potent pressor octapeptide, angiotensin II. Angiotensin II then causes constriction of the arterioles and is also believed to stimulate release of the sodium-retaining hormone, aldosterone, from the adrenal gland, thereby causing a rise in extracellular fluid volume. Thus, the renin-angiotensin system plays an important role in normal cardiovascular homeostasis and in some forms of elevated blood pressure (hypertension).

Inhibitors of angiotensin I converting enzyme have proven useful in the modulation of the renin-angiotensin system. Consequently, specific inhibitors of the catalytic and rate-limiting enzymatic step that ultimately regulates angiotensin II production, the action of renin on its substrate, have also been sought as effective investigative tools, as well as therapeutic agents in the treatment of hypertension and congestive heart failure.

Renin antibody, pepstatin, phospholipids, and substrate analogs, including tetrapeptides and octa- to tridecapeptides, with inhibition constants ($K_i$) in the $10^{-3}$ to $10^{-6}$ M region, have been studied.

Umezawa et al., in J. Antibiot. (Tokyo) 23: 259-262, 1970, reported the isolation of a peptide, pepstatin, from actinomyces that was an inhibitor of aspartyl proteases such as pepsin, cathepsin D, and renin. Gross et al., Science 175:656, 1972, reported that pepstatin reduces blood pressure in vivo after the injection of hog renin into nephrectomized rats. However, pepstatin has not found very wide application as an experimental agent because of its limited solubility and its inhibition of a variety of other acid proteases in addition to renin.

Many efforts have been made to prepare a specific renin inhibitor based on pig renin substrate analogy, since such analogy has been shown to correlate well with and predict human renin inhibitor activity. The octapeptide amino acid sequence extending from histidine-6 through tyrosine-13

```
 6   7   8   9   10  11  12  13
(-His-Pro-Phe-His-Leu-Leu-Val-Tyr-)
```

has been shown to have kinetic parameters essentially the same as those of the full tetradecapeptide renin substrate.

Kokubu et al., Biochem. Pharmacol., 22, 3217-3223, 1973, synthesized a number of analogs of the tetrapeptide found between residues 10 to 13, but while inhibition could be shown, inhibitory constants were only of the order of $10^{-3}$M. Analogs of a larger segment of renin substrate have been also synthesized, e.g., Burton et al., Biochemistry 14: 3892-3898, 1975, and Poulsen et al., Biochemistry 12: 3877-3882, 1973, but a lack of solubility and weak binding (large inhibitory constant) generally resulted.

Modifications to increase solubility soon established that the inhibitory properties of the peptides are markedly dependent on the hydrophobicity of various amino acid residues. These modifications also established that increasing solubility by replacing lipophilic amino acids with hydrophilic isosteric residues can become counter-productive. Other approaches to increasing solubility have also had limited success.

Modifications designed to increase binding to renin have also been made, but here too, with mixed

results.

A series of inhibitors of renin have been disclosed which contain the unnatural amino acid, statine or its analogs: see, e.g., Veber et al, U.S. Patents 4,384,994 and 4,478,826; Evans et al, U.S. Patent 4,397,786; Boger et al, Nature, 1983, 303, 81-84 and U.S. Patents 4,470,971; 4,485,099; 4,663,310 and 4,668,770; Matsueda et al, EP-A 128 762, 152 255; Morisawa et al., EP-A 186 977; Riniker et al, EP-A 111 266; Bindra et al, EP-A 155 809: Stein et al, Fed. Proc. 1986, 45, 869; and Hölzemann et al, German Offenlegungsschrift DE 3438545. Attempting to explain the effect of statine, Powers et al., in Acid Proteases, Structure, Function and Biology, Plenum Press, 1977, 141-157, observed that in pepstatin, statine occupies the space of the two amino acids on either side of the cleavage site of a pepsin substrate and Tang et al., in Trends in Biochem. Sci., 1:205-208 (1976) and J. Biol. Chem., 251:7088-94, 1976, suggested that the statine residue of pepstatin resembles the transition state for pepsin hydrolysis of peptide bonds.

Renin inhibitors containing other peptide bond isosteres, including a reduced carbonyl isostere have been disclosed by M. Szelke et al, in work described in published European Patent Applications 45 665 and 104 041; in U.S. Patent 4,424,207, and in PCT Int. Appl. WO 84/03044; in Nature, 299, 555 (1982); Hypertension, 4, Supp. 2, 59, 1981; and British Patent 1,587,809. In Peptides, Structure and Function: Proceedings of the Eighth American Peptide Symposium, ed. V. J. Hruby and D. H. Rich, p. 579, Pierce Chemical Co., Rockford, IL., 1983, Szelke et al also showed isosteric substitutions at the Leu-Leu site of cleavage, resulting in compounds with excellent potency. These and other peptide bond isosteres have also been disclosed in Buhlmayer et al in EP-A 144 290 and 184 550; Hester et al, EP-A 173 481; Raddatz, EP-A 161 588; Dann et al, Biochem. Biophys. Res. Commun. 1986, 134, 71-77; Fuhrer et al, EP-A 143 746; Kamijo et al, EP-A 181 110; Thaisrivongs et al, J. Med. Chem., 1985, 28, 1553-1555; Ryono et al., EP-A 181 071; and Evans et al, U.S. Patent 4,609,641.

Other modifications which have been tried include preparing renin inhibitors with non-peptide C-termini, such as disclosed in European Published Applications 172 346 and 172 347; Evans et al, J. Med. Chem., 1985, 28, 1755-1756; and Bock et al, Peptides, Structure and Function: Proceedings of the Ninth American Peptide Symposium, ed. C. M. Deber et al, pp.751-754, Pierce Chemical Co., Rockford, IL, 1985. Kokubu et al, in Hypertension, 1985, 7, Suppl. I, p. 8-10 and Matsueda et al, in Chemistry Letters, 1985, 1041-1044 and in European Published Applications 128 762 and 152 255 disclosed peptide aldehyde renin inhibitors, and Hanson et al in Biochem. Biophys. Res. Commun. 1985, 132, 155-161, reported peptide glycol inhibitors.

These various renin inhibitors all generally comprise peptide-based inhibitors in which a sequence of the type: ...A-B-D-E-F-G-J-K L... , where G is a peptide bond mimic and A,B,D,E,F,J,K, and L may individually be absent or may represent naturally-occuring or modified amino acids. Typical sequences of this type include:

```
      7   8   9   10  11  12
...BOC-Pro-Phe-His-Sta-Leu-Phe... ,
```

or

```
      8   9   10  11
...BOC-Phe-His-Sta-Leu... ,
```

where the N-terminus typically comprises an amino acid protecting group such as BOC or CBZ, and the N-terminal amino acids are Pro-Phe-His or Phe-His.

It was an object of this invention to prepare lower molecular weight peptides which have enhanced biological potency in inhibiting the renin enzyme. It was also an object to prepare peptides wherein a stabilizing peptide bond mimic is substituted for the 10- and 11- position amino acids in the analogous substrate. It was a further object of this invention to prepare peptides comprising modifications at the C-terminus which constuctively alter the solubility and bioavailability of the peptide. It was an additional object of this invention to prepare peptides which have greater oral bioavailability and increased duration of action.It was still a further object of this invention to prepare novel peptides which are more useful antihypertensive agents, and are compounds useful in treating hyperaldosteronism and congestive heart failure.

DETAILED DESCRIPTION OF THE INVENTION

The present invention discloses renin-inhibitory tetrapeptides of the formula:

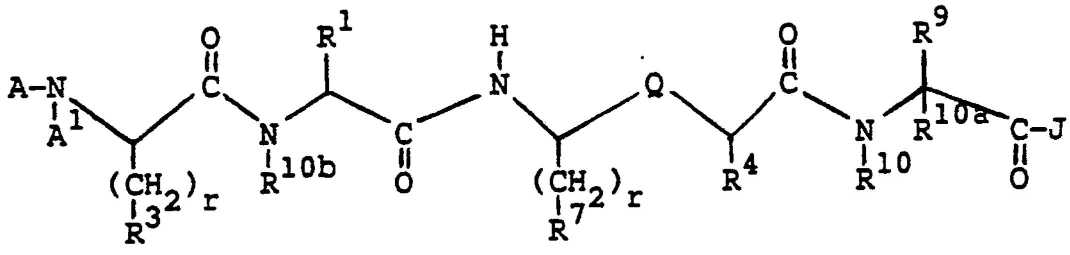

(I),

wherein:

A is hydrogen;

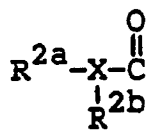

,

where

X is -O-;-O- CH-; - CH-O-; - CH-; N -; -NH- CH-; or-S- CH-; and

$R^{2a}$ and $R^{2b}$ and are independently hydrogen, or unsubstituted or mono or disubstituted $C_1$-$C_4$-alkyl, wherein the substituent(s) is/are independently selected from the group consisting of amino; mono- or di-$C_1$-$C_4$- alkylamino; t-butoxycarbonylamino; acetylamino; carbobenzyloxyamino; trimethylacetylamino; t-butylacetylamino; hydroxy; $-SO_3H$; $CO_2H$;$-CO_2$-$C_1$-$C_4$-alkyl; $C_1$-$C_4$-alkyl-$SO_2$; aryl, where aryl is unsubstituted or mono-, di-or trisubstituted phenyl or naphthyl, wherein the substituent(s) is/are independently selected from the group consisting of $C_1$-$C_8$-alkyl, amino, phenyl-$C_1$-$C_4$-alkyl, mono- or di-$C_1$-$C_4$-alkylamino, amino-$C_1$-$C_4$-alkyl, hydroxy-$C_1$-$C_4$-C4-alkyl, mono- or di-$C_1$-$C_4$-alkylamino-$C_1$-$C_4$-alkyl, guanidyl, guanidyl-$C_1$-$C_4$-alkyl, hydroxyl, $C_1$-$C_4$-alkoxy, $CF_3$, halo, CHO, $CO_2H$, $CO_2$-$C_1$-$C_4$-alkyl, $CO_2$-$C_1$-$C_4$-alkoxy, $NR^5R^6$, and $N(R^5)_3^{\oplus} A^{\ominus 4}$, wherein $R^5$ and $R^6$ are independently hydrogen, unsubstituted or monosubstituted $C_1$-$C_4$-alkyl, where the substituent is amino, mono- or di-$C_1$-$C_4$-alkylamino, hydroxyl, $C_1$-$C_4$-alkoxy or $N(C_1$-$C_4$-alkyl$)_3^{\oplus} A^{\ominus}$; and $A^{\ominus}$ is a counterion selected from the group consisting of single negatively-charged ions, such as chloride, bromide, nitrate, perchlorate, benzoate, maleate, benzene sulfonate, tartrate, hemitartrate and acetate; $-CONR^5R^6$, where $R^5$ and $R^6$ are as defined above; and Het, where Het is an unsubstituted or mono- or disubstituted 5-to-7-membered mono- or bicyclic or 7-to-10-membered bicyclic heterocyclic ring, wherein the one or two heteroatoms are independently selected from the group consisting of N, O, S, NO, SO, $SO_2$ and quaternized N, and the substituent(s) is/are independently selected from the group consisting of hydroxy, thiol, $C_1$-$C_6$-alkyl, $CF_3$, $C_1$-$C_4$-alkoxy, halo, aryl, as defined above, aryl-$C_1$-$C_4$-alkyl, amino, mono- or di-$C_1$-$C_4$-alkylamino, amino-$C_1$-$C_4$-alkyl, hydroxy-$C_1$-$C_4$-alkyl, di-$C_1$-$C_4$-alkylamino-$C_1$-$C_4$-alkyl, guanidyl, guanidyl-$C_1$-$C_4$-alkyl, $CO_2H$, $CO_2$-$C_2$-$C_4$-alkyl, $NR^5R^6$ and $N(R^5)_3^{\oplus} A^{\ominus}$, wherein $R^5$, $R^6$, and $A^{\ominus}$ are as defined above; or alternatively when N is present as a heteroatom, the substituents are $-(CH_2)_q$- or --$(CH_2)_2$-O-$(CH_2)_2$- and form a quaternary spirocyclic ring with the N-atom, wherein q is 3-to-6;

except that where X is-O-, only one of $R^{2a}$ or $R^{2b}$ is present;

$R^{2c}SO_2$-,

where

$R^{2c}$ is unsubstituted, mono- or di-substituted $C_1$-$C_4$ alkyl, wherein the substituent(s) is/are independently selected from the group consisting of amino, mono- of di-$C_1$-$C_4$-alkylamino, t-butoxycarbonylamino, carbobenzyloxyamino, acetylamino, trimethylacetylamino, hydroxy, $-SO_3H$, $-CO_2H$,$-CO_2$-$C_1$-$C_4$-alkyl, $C_1$-$C_4$-alkyl-$SO_2$- and Het, as defined above; or

Het-CO, where Het is as defined above;

$A^1$ is hydrogen or $C_1$-$C_4$-alkyl;

r is 1-to-4;

$R^3$ is hydrogen, $C_1$-$C_4$-alkyl, aryl, as defined above, aryl-$C_1$-$C_4$-alkyl, or indolyl;

$R^1$ is is hydrogen;
aryl, as defined above;
Het-$SO_t$-$CH_2$, where t is 0-to-2; or unsubstituted or monosubstituted $C_1$-$C_4$-alkyl, or $C_1$-$C_4$-alkylthio-$C_1$-$C_4$-alkyl, where the substituent is on the terminal carbon atom and is chosen from the group consisting of $C_1$-$C_4$-alkoxy, amino, mono-or di-$C_1$-$C_4$-alkylamino, carboxy, $CO_2$-$C_1$-$C_4$-alkyl, aryl, as defined above, Het, as defined above, or -$CONR^5R^6$ wherein $R^5$ and $R^6$ are as defined above;
$R^7$ is $C_3$-$C_6$-alkyl;
aryl, as defined above; or unsubstituted or mono-, di- or trisubstituted $C_3$-$C_7$-cycloalkyl, wherein the substituent(s) is/are independently selected from the group consisting of $C_1$-$C_4$-alkyl, trifluoromethyl, hydroxy, $C_1$-$C_4$-alkoxy and halo;
Q is

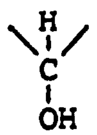 ; 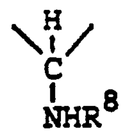 ; 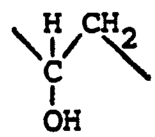 ; or 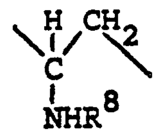 ,

where
$R^8$ is $C_1$-$C_4$-alkyl; formyl; $C_1$-$C_4$-alkanoyl; aroyl; $C_1$-$C_4$-alkoxycarbonyl; aryloxycarbonyl; or aryl-$C_1$-$C_4$-alkoxycarbonyl, wherein aryl is as defined above;
$R^4$ is hydrogen;
$C_1$-$C_8$-alkyl;
$C_2$-$C_8$-alkenyl;
mono- or disubstituted $C_2$-$C_8$-alkyl, wherein the substituent(s) is/are on the final 1 or/and 2 carbon atoms of the alkyl chain and is/are independently selected from the group consisting of hydroxy, carboxy, $CO_2$-$C_1$-$C_4$-alkyl, amino, mono- , di- or trialkylamino, guanidyl, $NR^5R^6$ and $N(R^5)_3^{\oplus} A^{\ominus}$, where $R^5$, $R^6$ and $A^{\ominus}$ are as defined above;
aryl, as defined above; or unsubstituted, mono-, di- or trisubstituted $C_3$-$C_7$-cycloalkyl, wherein the substituent(s) is/are independently selected from the group consisting of $C_1$-$C_4$-alkyl, trifluoromethyl, hydroxy, $C_1$-$C_4$-alkoxy and halo;
$R^9$ is -$(CH_2)_s$-$NR^{11}R^{12}$, where $R^{11}$ and $R^{12}$ are independently hydrogen; Het, as defined above; aryl, as defined above; $C_3$-$C_7$-cycloalkyl; or unsubstituted or monosubstituted $C_1$-$C_4$-alkyl, where the substituent is on the terminal carbon atom and is chosen from the group consisting of aryl, as defined above; Het, as defined above; amino; hydroxyl; mono- or di-$C_1$-$C_4$-alkylamino; $CO_2H$; $CONR^5R^6$, wherein $R^5$ and $R^6$ are as defined above; and $SO_2H$; and s is 1-to-5; -$(CH_2)_tN(R^{11})_3^{\oplus} A^{\ominus}$, where t is 2-to-5; and $R^{11}$ and $A^{\ominus}$ are as defined above;

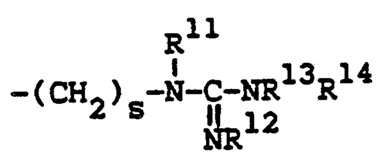 ,

where $R^{13}$ and $R^{14}$ are independently chosen from the definitions of $R^{11}$, or together are -$(CH_2)_4$- or -$(CH_2)_5$-; and $R^{11}$, $R^{12}$ and s are as defined above; -$(CH_2)_5N{=}CH$-$NR^{13}R^{14}$, where s, $R^{13}$ and $R^{14}$, or $R^{13}$ and $R^{14}$ taken together, are as defined above; or $(CH_2)_s$-$R^{18}$,
where
$R^{18}$ is -$CH(CH_2CH_2)_2NR^{10}$,

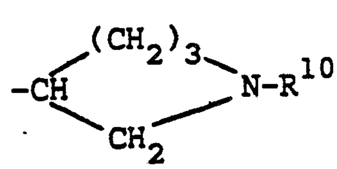

or

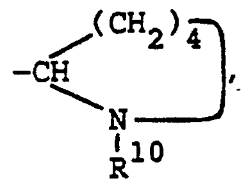

wherein $R^{10}$ is H or $C_1$-$C_4$-alkyl; and s is as defined above;
$R^{10a}$ and $R^{10b}$ are independently H or $C_1$-$C_4$-alkyl;

J is -NH- $\overset{R^{15}}{\overset{|}{C}}H$ $-(CH_2)_u$-$R^{16}$, where $R^{15}$ is hydrogen, $C_1$-$C_4$-alkyl or aryl-$C_1$-$C_4$-alkyl, wherein aryl is as defined above; u is 0-to-4; and $R^{16}$ is Het, as defined above; monosubstituted phenyl or naphthyl, wherein the substituent is selected from the group consisting of $C_1$-$C_8$-alkyl, amino, mono- or di-$C_1$-$C_4$-alkylamino, amino-$C_1$-$C_4$-alkyl, hydroxy-$C_1$-$C_4$-alkyl, mono-or di-$C_1$-$C_4$-alkylamino-$C_1$-$C_4$-alkyl, guanidyl, guanidyl-$C_1$-$C_4$-alkyl, $CF_3$, halo, CHO, $CO_2H$, $CO_2$-$C_1$-$C_4$-alkyl, $CO_2$-$C_1$-$C_4$-alkoxy, $NR^5R^6$ and $N(R^5)^{\oplus}_3 A^{\ominus}$, where $R^5$, $R^6$ and $A^{\ominus}$ are as defined above; or, when u is not equal to 0, also alternatively, $-NR^{11}R^{12}$ or $-N(R^{11}R^{12}R^{13})^{\oplus}A^{\ominus}$, wherein $R^{11}$, $R^{12}$, $R^{13}$ and $A^{\ominus}$ are as defined above;

-O- $\overset{R^{15}}{\overset{|}{C}}H$ $-(CH_2)_u$-$R^{16}$, where $R^{15}$, $R^{16}$ and u are as defined above;

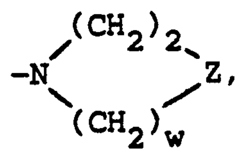

where Z is $-NR^{11}$, wherein $R^{11}$ is as defined above;

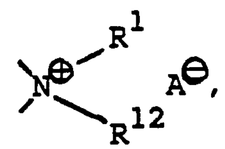

wherein $R^1$, $R^{12}$ and $A^{\ominus}$ are as defined above; O; S; SO; or $SO_2$; and w is 2 or 3;
-NH-Het, where Het is as defined above;

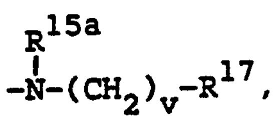

where v is 0-to-4; $R^{17}$ is Het, as defined above; aryl, as defined above; $C_1$-$C_4$-alkyl; or, when v is not equal to 0 or 1, alternatively $NH_2$, $NR^{11}R^{12}$, $N(R^{11}R^{12}R^{13})^{\oplus}A^{\ominus}$, hydroxyl, or $SO_3H$, wherein $R^{11}$, $R^{12}$, $R^{13}$, and $A^{\ominus}$ are as defined above; and $R^{15a}$ is $C_1$-$C_4$-alkyl or aryl-$C_1$-$C_4$-alkyl, wherein aryl is defined above; or when v is not equal to 0, also alternatively $CO_2H$, $CO_2$-$C_1$-$C_4$-alkyl or $CONR^5R^6$, wherein $R^5$ and $R^6$ are as defined above;

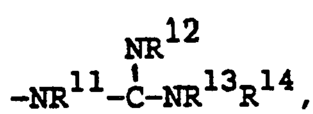

where $R^{11}$, $R^{12}$, $R^{13}$ and $R^{14}$ are as defined above; or $-N=CH-NR^{11}R^{12}$, where $R^{11}$ and $R^{12}$ are as defined above;
and pharmaceutically-acceptable salts thereof.

In the peptides of the present invention, the amino acid, A and J components have asymmetric centers and occur as racemates, racemic mixtures and as individual diastereomers, with all isomeric forms generally being included in the present invention. In particular, asymmetric carbon atoms in the dipeptidal analogous 10, 11 position substituent have either an S or an R configuration, and all other asymmetric carbon atoms have an S configuration, with preferred chiralities indicated in the following further description.

When any variable (e.g., aryl, Het, $R^5$, $R^6$, $R^{11}$, $R^{12}$, $R^{13}$, $R^{14}$, $A^{\ominus}$, etc.) occurs more than one time in any constituent or in formula I, its definition on each ocurrence is independent of its definition at every other occurrence.

As used herein, "alkyl" is intended to include both branched and straight-chain saturated aliphatic hydrocarbon groups having the specified number of carbon atoms (Me is methyl, Et is ethyl, Pr is propyl, Bu is butyl); "alkoxy" represents an alkyl group of indicated number of carbon atoms attached through an oxygen bridge; and "$C_3$-$C_7$-cycloalkyl" is intended to include saturated ring groups, such as cyclopropyl, cyclobutyl, cyclopentyl, cyclohexyl (Cyh) and cycloheptyl. "Alkanoyl" is intended to include those alkylcarbonyl groups of specified number of carbon atoms, which are exemplified by formyl, acetyl, propanoyl and butanoyl; "alkenyl" is intended to include hydrocarbon chains of either a straight- or branched- configuration and one unsaturation, which may occur at any point along the chain, such as ethenyl, propenyl, butenyl, pentenyl, and the like, and includes E and Z forms, where applicable; and "arylalkyl" represents aryl groups as herein defined which are attached through a straight-or branched- chain alkyl group of specified number of carbon atoms, such as, for example, benzyl, phenethyl, 3,3-diphenylpropyl, 3-indolymethyl, and the like. "Halo", as used herein, means fluoro, chloro, bromo and iodo, and "counterion" is used to represent a small, single negatively-charged specie, such as chloride, bromide, acetate, perchlorate, benzoate, amleate, benzene sulfonate, tartrate, hemitartrate and the like.

As used herein, with exceptions as noted, "aryl" is intended to mean phenyl (Ph) or naphthyl, which is optionally-substituted by from one- to three- members independently selected from the group consisting of amino (Am), mono- or di-$C_1$-$C_4$-alkylamino, amino-$C_1$-$C_4$-alkyl, hydroxy -$C_1$-$C_4$-alkyl, di-$C_1$-$C_4$-alkylamino-$C_1$-$C_4$-alkyl, guanidyl, guanidyl-$C_1$-$C_4$-alkyl, hydroxyl, $C_1$-$C_4$-alkoxy, $CF_3$, halo, CHO, $CO_2H$, $CO_2R^7$, $NR^5R^6$, wherein $R^7$, $R^5$ and $R^6$ are as defined above, or $N(R^5)_3^{\oplus} A^{\ominus}$, wherein $R^5$ is as defined above and $A^{\ominus}$ is a counterion, as defined herein. "Aroyl" is intended to include those aryl-carbonyl groups which are exemplified by benzoyl and naphthoyl.

The term "Het", as used herein, represents a 5- to 7-membered monocyclic or 7- to 10-membered bicyclic heterocyclic ring which is either saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. Fully-saturated heterocyclic substituents are preferred and those in which nitrogen is the heteroatom are also preferred, with those containing a single nitrogen atom being particularly preferred. In the case of a heterocyclic ring containing one or more nitrogen atoms, the point of attachment may be at one of the nitrogen atoms, or at any carbon atom. Examples of such heterocyclic elements include piperidyl, piperidinyl, piperazinyl, 2-oxopiperazinyl, 2-oxopiperidinyl, 2-oxopyrolodinal, pyrryl, pyrrolinyl, 4-piperidonyl, pyrrolidinyl, pyrazolyl, pyrazolinyl, pyrazolidinyl, imidazolyl, imidazolinyl, imidazolidinyl, pyridyl, pyrazinyl, pyrimidinyl, pyridazinyl, oxaolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, benzothiazolyl, benzoxazolyl, furyl, thienyl, benzothienyl, azepinyl, 2-oxoazepinyl, thiamorpholinyl, thiamorpholinyl sulfoxide, and thiamorpholinyl sulfone. The heterocyclic moiety is further optionally-substituted by from one- to four-members independently selected from the group consisting of hydroxyl, thiol, $C_1$-$C_6$-alkyl, $CF_3$, $C_1$-$C_4$-alkoxy, halo, aryl, aryl-$C_1$-$C_4$-alkyl, amino, mono- or di-$C_{1-4}$-alkylamino, amino-$C_1$-$C_4$-alkylamino, amino-$C_1$-$C_4$-alkyl, hydroxy-$C_1$-$C_4$-alkyl, $C_1$-$C_4$-dialkylamino-$C_1$-$C_4$-alkyl, guanidyl, guanidyl $C_1$-$C_4$-alkyl, hydroxyl, CHO, $CO_2H$, $CO_2R^7$, $NR^5R^6$, wherein $R^7$, $R^5$ and $R^6$ are as defined above, or $N(R^5)_3^{\oplus} A^{\ominus}$, wherein $R^5$ is as defined above and $A^{\ominus}$ is a counterion, as defined herein, with aryl-$C_1$-$C_4$-alkyl being preferred, or a spiro quaternary N bicyclic may be formed.

The following additional abbreviations have also been used herein:

| Abbreviated Designation | Amino Acid/Residue |
|---|---|
| ACHPA | (3S,4S)-4-amino-5-cyclohexyl-3-hydroxypentanoic acid |
| Arg | D- or L-arginine |
| Cal (Cha) | 3-cyclohexylalanine |
| Gly | glycine |
| His | D- or L-histidine |
| HomoACHPA (H-ACHPA) | homologated ACHPA |
| HArg | D- or L- homoarginine |
| HPhe | D- or L-homologated phenylalanine (homophenylalanine) |
| HLys | D- or L-homolysine |
| Ile | D- or L-isoleucine |

| | |
|---|---|
| Leu | D- or L-leucine |
| Lys | D- or L-lysine |
| Nle | D- or L-norleucine |
| Nva | D- or L-norvaline |
| Orn | D- or L-ornithine |
| Phe | D- or L-phenylalanine |
| Sta | statine, (3S,4S)-4-amino-3-hydroxy-6-methylheptanoic acid |
| | **Protecting Group** |
| BOC (Boc) | t-butyloxycarbonyl |
| CBZ (Cbz or Z) | benzyloxycarbonyl(carbobenzoxy) |
| DNP | 2,4-dinitrophenyl |
| EtOC | ethoxycarbonyl |
| OEt | ethoxy, except when it immediately follows an amino acid residue abbreviation and it represents ethyl ester |
| TBDMS | t-butyldimethylsilyl |
| | **Activating Group** |
| HOBt(HBT) | N-hydroxybenzotriazole hydrate |
| | **Condensing Agent** |
| DCC | N,N'-dicyclohexylcarbodiimide |
| | **Reagent** |
| $i\text{-}Pr_2NEt$ | diisopropylethylamine |
| $TEA(Et_3N)$ | triethylamine |
| TFA | trifluoroacetic acid |

## Coupling Reagents

| | |
|---|---|
| BOP reagent | benzotriazol-1-yloxytris(dimethyl-amino)phosphonium hexafluoro-phosphate |
| BOP-Cl | bis(2-oxo-3-oxazolidinyl)phosphinic chloride |

## Solvent

| | |
|---|---|
| AcOH (HOAc) | acetic acid |
| $CH_2Cl_2$ | methylene chloride |
| DMF | N,N-dimethylformamide |
| EtOAc (E) | ethyl acetate |
| THF | tetrahydrofuran |

The novel renin inhibitory peptides of the present invention may alternately be simplified and described in terms of common amino acid components and closely related analogs thereof, in accordance with the following formula, analogous to formula I: A-B-D-G-T-J (II), wherein

A is Boc, EtOC, isopropylsulfonyl, (2-N-Boc-amino)ethyloxycarbonyl, (5-carboxy)pentanoyl or (morpholin-4-yl)carboxyl;
B is Phe, HPhe, Trp or Tyr(OMe);
D is His, Ala, Ile, Leu, Met, Nle, Nva or Phe;
G is Sta, ACHPA, HomoACHPA, AmACHPA, or HomoAmACHPA;
T is Arg, HArg, Orn, Dap, ($N^{\alpha}$-Me)Lys or Lys; and
J is 4-aminomethylpyridine, 4-(N-methyl)aminomethylpyridine, 3-amino-2-oxoazepine or 4-aminomethylpiperidine.

In terms of substrate analogy, a unique aspect and essential feature of the present invention is the substitution of the G-component for the double amino acid sequence, $Leu^{10}$-$Leu^{11}$ in the endogenous pig renin substrate, or $Leu^{10}$-$Val^{11}$ in the endogenous human renin substrate

| 7 | 8 | 9 | 10 | 11 | 12 | 13 |
|---|---|---|---|---|---|---|
| (Pro | Phe | His | Leu | Val | Ile | His) , |

which substitution for both amino acids at the cleavage site rather than just one is believed to result in an improved substrate analogy and better fit to the renin enzyme in linear extent.

In addition to an increase in metabolic stability achieved with the substitution of statine analogs/residue (particularly ACHPA), and in the shortening of the peptide chain, the novel peptides of the instant invention successfully use hydrophilic and/or hydrophobic groups strategically and comprise simplified N- and C-terminii, preferably with the elimination of α-amino acids following ACHPA at the C-terminus of these peptides. These peptides are of lower molecular weight and different structure, with an enhanced potency and duration of action, than known peptides of this class.

It will be understood that closely-related analogs of the above common amino acids, for example, aliphatic amino acids in addition to Ala, Val, Leu, and Ile, such as α-aminobutyric acid (Abu), and substituted phenyl derivatives of Phe, are included in the broad description of the novel inhibitory peptides of the present invention represented by Formulae I and II and related definitions.

Preferred renin-inhibitory peptides are those wherein, in structures according to formula II, A is Boc, $Boc\text{-}NHCH_2CH_2OCO$, EtOC, $i\text{-}PrSO_2$, morpholino-CO or $HO_2(CH_2)_4CO$; B is Phe; D is His; G is ACHPA or HomoACHPA; T is Lys, Orn, Dap or ($N^{\alpha}$-Me)Lys; and

J is 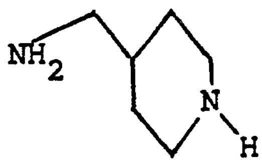 , 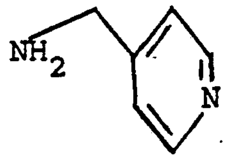 , 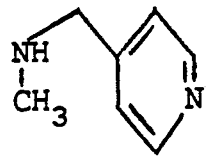 ,

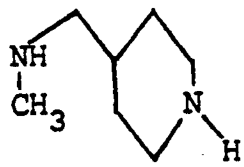 .

Representative preferred renin-inhibitory peptides of the present invention include the following:

Boc-Phe-His-ACHPA-Lys-$NHCH_2$(pyridin-4-yl)
Boc-Phe-His-ACHPA-Lys-$NHCH_2$(pyridin-2-yl)
Boc-Phe-His-ACHPA-($N^{\alpha}$-Me)Lys-$NHCH_2$(pyridin-4-yl)
Boc-Phe-His-ACHPA-($\alpha$-Me)Lys-$NHCH_2$(pyridin-4-yl)
Boc-Phe-His-ACHPA-Lys-$N(CH_3)CH_2$(-pyridin-4-yl)
Boc-Phe-His-ACHPA-($N^{\alpha}$-Me)Lys-$N(CH_3)CH_2$(-pyridin-4 yl)
Boc-Phe-His-ACHPA-Lys-$NHCH_2$(piperidin-4-yl)
Boc-Phe-His-ACHPA-Lys-$NHCH_2$(piperidin-2-yl)
Boc-Phe-His-ACHPA-(-$N^{\alpha}$-Me)Lys-$NHCH_2$(piperidin-4-yl)
Boc-Phe-His-ACHPA-($\alpha$-Me)Lys-$NHCH_2$(piperidin-4-yl)
Boc-Phe-His-ACHPA-Lys-$N(CH_3)CH_2$(piperidin 4-yl)
Boc-Phe-His-ACHPA-Lys-$NHCH_2$(1-methylpiperidin-4-yl)
Boc-Phe-His-ACHPA-Lys-$NHCH_2$(1-benzylpiperidin-4-yl)
Boc-Phe-His-ACHPA-Lys-$NHCH_2$(1-carboxymethylpiperidin-4-yl)
Boc-Phe-His-ACHPA-($N^{\alpha}$-Me)Lys-$N(CH_3)CH_2$(piperidin-4-yl)
[Boc-Phe-His-ACHPA-Lys-$NHCH_2$(1-methylpyridin-4-yl)]$^{\oplus}$ OAc$^{\ominus}$
Boc-Phe-His-ACHPA-($N^{\epsilon}$,$N^{\epsilon}$-dimethyl)Lys-$NHCH_2$ (pyridin-4-yl)
Boc-Phe-His-ACHPA-($N^{\epsilon}$-isopropyl)Lys-$NHCH_2$(pyridin-4-yl)
Boc-Phe-His-ACHPA-Orn-$NHCH_2$(pyridin-4-yl)
Boc-Phe-His-ACHPA-$NH(CH_2)CO$-$NHCH_2$(pyridin-4-yl)
Boc-Phe-His-ACHPA-($N^{\epsilon}$,$N^{\epsilon}$)-Lys-$N(CH_3)$ $CH_2$(pyridin-4-yl)
Boc-Phe-His-ACHPA-Lys-NH(3-quinuclidinyl)
[Boc-Phe-His-ACHPA-Lys-NH(N-methyl-3-quinuclidinyl)]$^{\oplus}$ Cl$^{\ominus}$
[Boc-Phe-His-ACHPA-($N^{\epsilon}$, $N^{\epsilon}$, $N^{\epsilon}$-trimethyl)-Lys-$NHCH_2$(pyridin-4-yl]$^{\oplus}$Cl$^{\ominus}$
Boc-Phe-His ACHPA-($N^{\epsilon}$-benzyl)Lys-$NHCH_2$(pyridin-4-yl)
Boc-(p-OMe)Phe-His-ACHPA-Lys-$NHCH_2$(pyridin-4 yl)
Boc-(p-OMe)Phe-His-ACHPA-Lys-NH(3-quinuclidinyl)
Boc-(p-OMe)Phe-His-ACHPA-($N^{\epsilon}$,$N^{\epsilon}$-diethyl)-Lys-$NHCH_2$(pyridin-4-yl)
Boc-Phe-His-ACHPA-$NHCH[(CH_2)_4NHC(=NCH_3)NHCH_3]CO$-$NHCH_2$(pyridin-4-yl)
Boc-Phe-His-ACHPA-($N^{\epsilon}$-imidazolin-2-yl)Lys-$NHCH_2$(pyridin-4-yl)
Boc-Phe-His-ACHPA-Lys-$NHCH_2$(2-butyl)$CH_2N(CH_3)_3^{\oplus}$ OAc$^{\ominus}$
Boc-Phe-His-Cal[$CH(OH)CH_2$]Val-Lys-NHC-$_2$(pyridin-4-yl)
Boc-Phe-His-Cal[$CH(OH)CH_2$]Val-($\alpha$-Me)Lys-$NHCH_2$(pyridin-4-yl)
Boc-Phe-His-Cal[$CH(OH)CH_2$]Val-Lys-$N(CH_3)CH_2$(pyridin-4-yl)
Boc-Phe-His-Cal[$CH(OH)CH_2$]Val-Lys-$N(CH_2CH_2)_2O$
[Boc-Phe-His-Cal[$CH(OH)CH_2$]Val-Lys-$NHCH_2CH_2N(CH_2CH_2)_2O(CH_3)$]$^{\oplus}$Cl$^{\ominus}$
Boc-Phe-($N^{\alpha}$-Me)His-Cal[$CH(OH)CH_2$]Val-Lys-$NHCH_2$ (pyridin-4-yl)
Boc-Phe-($N^{\alpha}$-Me)His-Cal[$CH(OH)CH_2$]Val-($\alpha$-Me)Lys-$NHCH_2$(pyridin-4-yl)
Boc-Phe-($N^{\alpha}$-Me)His-Cal[$CH(OH)CH_2$]Val-Lys-$N(CH_3)$-$CH_2$(pyridin-4-yl)
Boc-Phe-($N^{\alpha}$-Me)His-Cal[$CH(OH)CH_2$]Val-Lys-$N(CH_2CH_2)_2O$
$HO_2C(CH_2)_4CO$-Phe-His-ACHPA-Lys-$NHCH_2$(pyridin-4-yl)

HO$_2$C(CH$_2$)$_4$CO-Phe-His-ACHPA-Lys-NHCH$_2$(piperidin-4-yl)
HO$_2$CCH$_2$C(CH$_3$)$_2$CO-Phe-His-ACHPA-Lys-NHCH$_2$(piperidin-4-yl)
O(CH$_2$CH$_2$)$_2$NCO-Phe-His-ACHPA-Lys-NHCH$_2$(pyridin-2-yl)
iPrSO$_2$-Phe-His-ACHPA-Lys-NHCH$_2$(pyridin-4-yl)
BocNHCH$_2$CH$_2$OCO-Phe-His-ACHPA-Lys-NHCH$_2$(pyridin-4-yl)
(CH$_3$)$_2$N(CH$_2$)$_3$CO-Phe-His-ACHPA-Lys-NHCH$_2$(pyridin-4-yl)
O(CH$_2$CH$_2$)$_2$NCO-(N$^{\alpha}$-Me)Phe-His-ACHPA-Lys-NHCH$_2$(pyridin-4-yl)
CH$_3$N(CH$_2$CH$_2$)$_2$NCO-Phe-His-ACHPA-Lys-NHCH$_2$(pyridin-2-yl)
SO$_2$(CH$_2$CH$_2$)$_2$NCO-Phe-His-ACHPA-Lys-NHCH$_2$(-pyridin-2-yl)
SO$_2$(CH$_2$CH$_2$)$_2$NCO-(N$^{\alpha}$-Me)Phe-His-ACHPA-Lys-NHCH$_2$(piperidin-4-yl)
HO$_2$CCH$_2$N(CH$_2$CH$_2$)$_2$NCO-Phe-His-ACHPA-Lys-NHCH$_2$(pyridin-4-yl)
HO$_2$CCH$_2$CH$_2$N(CH$_2$CH$_2$)$_2$NCO-Phe-His-ACHPA-Lys-NHCH$_2$(pyridin-4-yl)
HO$_2$CCH$_2$(CH$_3$)NCO-Phe-His-ACHPA-Lys-N(CH$_3$)CH$_2$(piperidin-4-yl)
O(CH$_2$CH$_2$)$_2$NCH$_2$CH$_2$SO$_2$-Phe-His-ACHPA-Lys-N(CH$_3$)CH$_2$-(pyridin-4-yl)
Boc-Phe-His-(2S-isobutyl)ACHPA-Lys-NHCH$_2$(pyridin-4-yl)
Boc-Phe-His-AmACHPA-Lys-NHCH$_2$(pyridin-2-yl)
Boc-Phe-His-(2S-allyl)ACHPA-(N$^{\alpha}$-Me)Lys-NHCH$_2$(pyridin-4-yl)
Boc-Phe-His-(2S-ethyl)ACHPA-($\alpha$-Me)Lys-NHCH$_2$(pyridin-4-yl)
Boc-Phe-His-(2S-n-butyl)ACHPA-Lys-N(CH$_3$)CH$_2$(pyridin-4-yl)
Boc-Phe-His-AmACHPA-(N$^{\alpha}$-Me)Lys-N(CH$_3$)CH$_2$(pyridin-4-yl)
Boc-Phe-His-AmACHPA-Lys-NHCH$_2$(pyridin-4-yl)
Boc-NHCH$_2$CH$_2$O-CO-Phe-His-ACHPA-Lys-NHCH$_2$(piperidin-4-yl)
O(CH$_2$CH$_2$)$_2$NCO-Phe-His-ACHPA-Lys-NHCH$_2$(piperidin-4-yl)
iPrSO$_2$-Phe-His-ACHPA-Lys-NHCH$_2$(piperidin-4-yl)

The pharmaceutically-acceptable salts of the peptides of Formula I (in the form of water- or oil-soluble or dispersible products) include the conventional non-toxic salts or the quaternary ammonium salts of these peptides, which are formed, e.g., from inorganic or organic acids or bases. Examples of such acid addition salts include acetate, adipate, alginate, aspartate, benzoate, benzene-sulfonate, bisulfate, butyrate, citrate, camphorate, camphor-sulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methane- sulfonate, 2- naphthalenesulfonate, nicotinate, oxalate, pamoate, pectinate, persulfate, 3-phenyl- propionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others.

Peptide renin inhibitors of Formula I may be prepared in accordance with well-known procedures for preparing peptides from their constitutent amino acids.

Structures and abbreviations for the G components of the renin-inhibitory peptides of the present invention include:

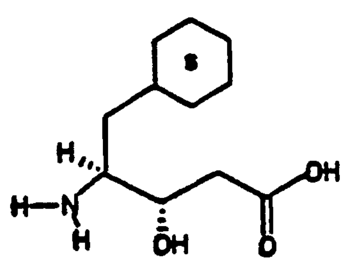

1. ACHPA

with BOC-ACHPA-OEt being prepared by the method described by Boger et al, J. Med. Chem 1985, 28, 1779-1790;

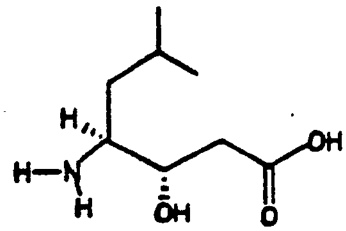

2. Statine (Sta)

with BOC-Sta-OEt being prepared in accordance with the procedure described by Rich et al, J. Org. Chem., 43, 3624(1978);

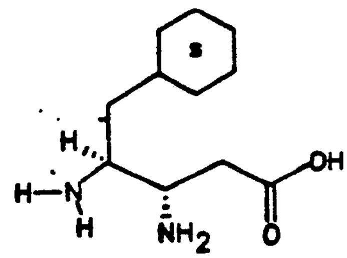

3. AmACHPA

with BOC-AmSta(CBZ)-OH being prepared as described by Jones et al, in Peptides, Structure and Function. Proceedings of the Ninth American Peptide Symposium (eds. C. M. Deber, V.J. Hruby and K. D. Kopple) pp. 759-62, 1985, Pierce Chemical Co., Rockford, IL.; Arrowsmith et al, J. Chem. Soc. Chem. Commun. 755-7 (1986); and Raddatz et al, Published Eur. Pat. Appl. 161,588; and BOC-AmACHPA(CBZ)OH being prepared in similar fashion;

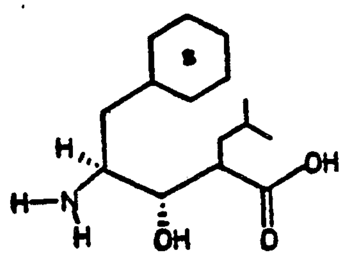

4. (2-isobutyl)ACHPA

with efficient methods for preparing the 2-substituted statine component G in a suitably protected form being described in Pub. Eur. Pat Appln 157,409 (with other pertinent references including D. Veber et al, Biochem. Soc. Trans., 12, 956-959 (l984) and H. Stein et al, Fed. Proc. 45, 869, Abstract No 4151 (1986);

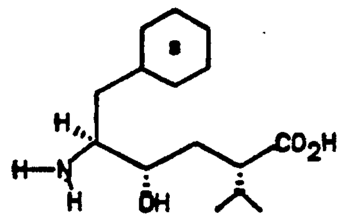

Cal[CH(OH)$CH_2$]Val

5.

with BOC-Cal[CH(OH)$CH_2$]Val-OH lactone being obtained from intermediates prepared using methods described by P. Buhlmayer et al, in Published European Patent Application 184,550-A2, and synthetic routes to similar peptide bond isosteres being described in the following:

a. Szelke et al, in Peptides, Structure and Function. Proceedings of the Eighth American Peptide Symposium (ed. V.J. Hruby and D.H. Rich) pp. 579-82, Pierce Chemical Co., Rockford, IL;

b. D.T. Pals et al in European Patent Appln. 173,481-A2;

c. B.E. Evans et al, J. Org. Chem., 50, 4615-1625 (1985);

d. A.H. Fray et al, J. Org. Chem., 51, 4828-4833 (1986);

e. M. Szelke et al, PCT Int. Appl. WO 84 03,044; and

f. D.J. Kempf, J. Org Chem., 51, 3921-3926 (1986); and

When the unit T consists of an $N^{\alpha}$-alkyl (for example, $N^{\alpha}$-methyl) amino acid, procedures well-known in peptide synthesis are used to prepare this amino acid and to couple it. In general, the mixed anhydride procedure (with pivaloyl chloride and N-methylmorpholine) is used, as illustrated by Wenger, Helv. Chem. Acta., 1984, 67, 502-525, or the BOP-Cl procedure of Rich et al., J. Amer. Chem. Soc., 1985, 107, 4342-43, is used. N-methyl amino acids may be prepared, for example, by the method of O'Donnell, Tetrahedron Letter, 1984, 25, 3651, while $N^{\alpha}$-alkyl amino acids generally may be prepared by the procedure of Freidinger, J. Org. Chem., 1983, 48, 77-81 or of Hansen et al., J. Org. Chem., 1985, 50, 945-950.

Peptides of Formula I which contain ACHPA as a peptide bond mimic are prepared using the following procedures or similar procedures (as explained below):

The peptide coupling reactions in the following Routes A, B, C, and D include those brought about by the action of DCC/HOBt (dicyclohexylcarbodiimide/N-hydroxybenzotriazole), DPPA (diphenylphosphorylazide), "BOP reagent" (benzotriazol-1-yloxytris (dimethylamino)phosphonium hexafluorophosphate), BOP-Cl (bis(2-oxo-3-oxazolidinyl)phosphinic chloride), DSO (N,N′ disuccinimidyl oxalate), and other well-known coupling reagents.

In these procedures, the amino acids represented by the elements B, D and T above are referred to as, respectively: $AA^2$, $AA^1$ and $AA^3$ and $R^{2a}$ and $R_{2b}$ are as defined above.

Route A:

Step A1:

BOC-$AA^3$ (1) is coupled with an amine component $R^{2c}R^{2d}NH$ which corresponds to the J element of formula I above, providing amide 2. Amide 2 is treated with anhydrous TFA, resulting in amide 3. Additional reactive functional groups in the amino component $R^{2c}R^{2d}NH$ are protected during the above steps (and those to follow below) with protecting groups, such as CBZ for amino groups, benzyl ethers for alcohols, and benzyl esters for carboxylic acids.

Step A2:

BOC-ACHPA-OEt (4) is treated with anhydrous TFA to remove the BOC protecting group, giving ACHPA-OEt (5).

Step A3:

Using standard methods, a dipeptide protected at the N-terminus with a BOC group (BOC-$AA^2$-$AA^1$) is coupled to 5, giving a coupled product 8. Alternatively, BOC-$AA^1$ is coupled to ACHPA-OEt giving 6. The coupled product 6 is treated with anhydrous TFA to removed the Boc protecting group, and the resulting product 7 is coupled with BOC-$AA^2$, giving 8.

Step A4:

The resulting coupled product 8 is treated with methanolic hydrazine, giving the corresponding hydrazide 9.

Step A5:

Hydrazide 9 is treated with acidic isoamyl nitrite, producing the corresponding acyl azide, which is treated in situ with 3, giving coupled product 10. Alternatively, coupled product 8 is treated with sodium hydroxide in THF-$H_2O$ and the resulting carboxylic acid derivative coupled with 3, giving 10.

Step A6:

The N-terminal BOC protecting group is then removed from 10 by treatment with anhydrous TFA, and the resulting tripeptide analog 11 treated with an acylating agent represented by $R^{2a}(R^{2b})$X-CO-W, where this acylating agent is an acid chloride, another activated carboxylic acid derivative or a carbamoyl chloride, sulfonyl chloride or chloroformate. This gives acylated tripeptide derivative 12.

Step A6a:

Alternatively, compound 12 may be prepared using the following sequence: An acylated amino acid $R^{2a}(R^{2b})$X-CO-$AA^2$ is coupled to compound 7, which is prepared as described in steps A2 and A3. The resulting coupled product 8a is then treated as described for 8 in steps A3 and A4, giving first hydrazide 9a, then after coupling to $R^{2c}R^{2d}$NH, the coupled product 12.

Step A7:

During the steps above, reactive functional groups present in the side chains of amino acid components or in the $R^{2a}R^{2b}$NH- element are protected with protecting groups. These may be CBZ groups for amines, benzyl ethers for alcohols, and benzyl esters for carboxylic acids. In the case of histidine, the BOC protecting may be used for protection of the side-chain imidazole ring; this group is removed in step A4 during the treatment with hydrazine, or alternatively in step A5 during treatment with sodium hydroxide. Thereafter the histidine imidazole ring may be left unprotected.

Step A8:

In cases where a quaternized amino group is to be introduced into the C-terminal $R^{2c}R^{2d}$NH-element of compound 13, one of the following procedures is followed to introduce the quaternized amine group:

Procedure 1: A tertiary amine within the $R_1$ or $R_2$ group of $R^{2c}R^{2d}$NH is quaternized by treatment of $R^{2c}R^{2d}$NH-BOC with an alkyl halide and $KHCO_3$ in methanol or ethanol, or with an alkyl halide in DMF. The resulting quaternary ammonium salt is then treated with anhydrous TFA to remove the BOC protecting group, and the resulting amine used for preparation of 3 as described above. Likewise an amino acid $AA^3$, the side-chain of which contains a quaternary ammonium group, may

be used for the preparation of 3.

Procedure 2: A compound 12, in which the $R^{2c}R^{2d}N$- element or the side-chain of $AA^3$ contains a teriary amine, is prepared and then the tertiary amine is quaternized by treatment with an alkyl halide in DMF. In this latter case, histidine, when present as the $AA^1$ element, must first be reprotected as the BOC derivative by treatment of 12 with di-t-butyldicarbonate. The BOC group is then removed after step A9 by treatment of 13 with $K_2CO_3$ in methanol or with anhydrous ammonia in DMF or methanol.

Step A9:

Protecting groups are removed from 12 by hydrogenolysis, giving 13.

| Compound | Formula |
|---|---|
| 1 | BOC-$AA^3$ |
| 2 | BOC-$AA^3$-$NR^{2c}R^{2d}$ |
| 3 | $AA^3$-$NR^{2c}R^{2d}$ |
| 4 | BOC-ACHPA-OEt |
| 5 | ACHPA-OEt |
| 6 | BOC-$AA^1$-ACHPA-OEt |
| 7 | $AA^1$-ACHPA-OEt |
| 8 | BOC-$AA^2$-$AA^1$-ACHPA-OEt |
| 8a | $R^2(R^{2b})$X-CO-$AA^2$-$AA^1$-ACHPA-OEt |
| 9 | BOC-$AA^2$-$AA^1$-ACHPA-$NHNH_2$ |
| 9a | $R^{2a}(R^{2b})$X-CO-$AA^2$-$AA^1$-ACHPA-$NHNH_2$ |
| 10 | BOC-$AA^2$-$AA^1$-ACHPA-$AA^3$-$NR^{2c}R^{2d}$ |
| 11 | $AA^2$-$AA^1$-ACHPA-$AA^3$-$NR^{2c}R^{2d}$ |
| 12(13) | $R^{2a}(R^{2b})$X-CO-$AA^2$-$AA^1$-ACHPA-$AA^3$-$NR^{2c}R^{2d}$ |

Route B:

Step B1:

BOC-ACHPA-OEt is treated with sodium hydroxide in THF-$H_2O$, giving BOC-ACHPA.

Step B2:

BOC-ACHPA is coupled with 3, giving coupled product 14.

Step B3:

Compound 14 is treated with anhydrous TFA to remove the BOC protecting group, giving 15.

Step B4:

Compound 15 is coupled with a dipeptide protected at the N-terminus with a BOC protecting group (BOC-$AA^2$-$AA^1$), giving coupled product 17. Alternatively, 15 is coupled with BOC-$AA^1$, giving 16. Treatment of 16 with anhydrous TFA and coupling of the resulting product with BOC-$AA^2$ gives 17.

Step B5:

The N-terminal BOC protecting group is then removed from 17 by treatment with anhydrous TFA, and the resulting tripeptide analog 18 treated with an acylating agent represented by $R^{2a}(R^{2b})X$-CO-W, where this acylating agent is an acid chloride, another activated carboxylic acid derivative or a carbamoyl chloride, sulfonyl chloride or chloroformate. This gives acylated tripeptide derivative 12.

Step B6:

In cases where a quaternized amino group is to be introduced into the C-terminal $R^{2c}R^{2d}N$-element of compound 13, one of the following procedures is followed to introduce the quaternized amine group:

Procedure 1: A tertiary amine within the $R_1$ or $R_2$ group of $R^{2c}R^{2d}NH$ is quaternized by treatment of $R^{2c}R^{2d}N$-BOC with an alkyl halide and $KHCO_3$ in methanol or ethanol, or with an alkyl halide in DMF. The resulting quaternary ammonium salt is then treated with anhydrous TFA to remove the BOC protecting group, and the resulting amine used for the preparation of 3 as described in route A.

Procedure 2: A compound 12, in which $AA^3$ or the $R^{2c}R^{2d}N$- element contains a teriary amine, is prepared and the tertiary amine is then quaternized by treatment with an alkyl halide DMF. In this latter case, histidine, when present as the $AA^1$ element, must first be reprotected as the BOC derivative by treatment of 12 with di-t-butyldicarbonate. The BOC group is then removed after step B7 by treatment of 13 with $K_2CO_3$ in methanol or with anhydrous ammonia in DMF or methanol.

Step B7:

As described above in route A, reactive functional groups in amino acid side chains of $AA^1$, $AA^2$, and $AA^3$, and in the $R^{2c}R^{2d}N$- component are protected during the coupling steps above. The protecting groups are now removed from coupled product 12 by hydrogenolysis giving compound 13.

| Compound | Formula |
|---|---|
| 14 | BOC-ACHPA-$AA^3$-$NR^{2c}R^{2d}$ |
| 15 | ACHPA-$AA^3$-$NR^{2c}R^{2d}$ |
| 16 | BOC-$AA^2$-ACHPA-$AA^3$-$NR^{2c}R^{2d}$ |
| 17 | BOC-$AA^2$-$AA^1$-ACHPA-$AA^3$-$NR^{2c}R^{2d}$ |
| 18 | $AA^2$-$AA^1$-ACHPA-$AA^3$-$NR^{2c}R^{2d}$ |

Peptides of Formula I which contain statine are prepared as described above in routes A and B except that BOC-ACHPA-OEt is replaced with BOC-Sta-OEt. In this way, peptides such as 19 may be prepared.

| | |
|---|---|
| 19 | $R^{2a}(R^{2b})X$-CO-$AA^2$-$AA^1$-Sta-$AA^3$-$NR^{2c}R^{2d}$ |

Peptides of Formula I which contain amino-ACHPA-(AmACHPA) or aminostatine (AmSta) are prepared as described above in Routes A and B except that BOC-ACHPA-OEt is replaced with BOC-AmACHPA-(CBZ)-OEt or with BOC-AmSta(CBZ)-OEt. The CBZ group of the AmACHPA or AmSta element is removed by hydrogenolysis step A9 or B7. In this way, peptide such as 20 (and the corresponding AmSta analogss) may be prepared.

| | |
|---|---|
| 20 | $R^{2a}(R^{2b})X$-CO-$AA^2$-$AA^1$-AmACHPA-$AA^3$-$NR^{2c}R^{2d}$ |

Peptides of Formula I which contain a 2-substituted statine or ACHPA element as a peptide bond mimic may be prepared as described above in Routes A and B except that BOC-ACHPA-OEt is replaced with a

suitably protected 2-substituted ACHPA or 2-substituted statine derivative, for example BOC-(2 allyl)-ACHPA-OEt or BOC-(2-isobutyl)Sta-OEt. In this way, peptides such as 21 may be prepared.

| | |
|---|---|
| 21 | $R^{2a}(R^{2b})$X-CO-$AA^2$-$AA^1$-(2-isobutyl)ACHPA-$AA^3$-$NR^{2c}R^{2d}$ |

Peptides of Formula I which contain Cal[CH(OH)$CH_2$]Val as a peptide bond mimic are prepared as described below in Routes C and D.

Route C:

Step C1:

The Boc protecting group is removed from lactone 22 by treatment with anhydrous TFA, giving lactone 23.

Step C2:

Lactone 23 is coupled with a Boc-protected amino acid (Boc-$AA^1$) giving 24 or with a N-terminal Boc-protected dipeptide (Boc-$AA^2$-$AA^1$) giving 25. Compound 25 may also be prepared by treating 24 with anhydrous TFA, and coupling the product with Boc-$AA^2$. Treatment of 24 (or 25) with anhydrous TFA, and coupling of the product with $R^{2a}(R^{2b})$X-CO-W, as defined above, yields 26 (or 27).

Step C3:

Lactone 26 is treated with aqueous potassium hydroxide to give the corresponding hydroxyacid. The hydroxyacid is treated with t-butyldimethylsilyl chloride and imidazole, then with HOAc in THF-$H_2O$, giving the protected hydroxyacid 27.

Step C4:

Compound 27 is coupled with 3, giving 28. Additional reactive functional groups in the amino acid side-chains or in amino component $R^{2c}R^{2d}$NH are protected with protecting groups such as CBZ for amino and guanidino groups, benzyl ethers for alcohols, and benzyl esters for carboxylic acids. When histidine is used as an amino acid component, the side-chain imidazole ring may be protected during step C2 with a BOC protecting group. This protecting group is removed during treatment with TFA, and the imidazole ring left unprotected during subsequent N-terminal coupling or acylation reactions. Alternatively histidine may be protected with a DNP protecting group during step C2. This group is then removed in step C3 during the potassium hydroxide treatment.

Step C5:

The silyl protecting group is removed from 28 by treatment with tetrabutylammonium fluoride in THF-DMF, giving 29.

Step C6:

In some cases, a quaternized amino group is present in the C-terminal $R^{2c}R^{2d}$N- element of compound 30. In this case, on the following procedures is used:

Procedure 1: A tertiary amine within $2^{2c}$ or $R^{2d}$ of $R^{2c}R^{2d}NH$ is quaternized by treatment of $R^{2c}R^{2d}N$-BOC with an alkyl halide and $KHCO_3$ in methanol or ethanol or with an alkyl halide in DMF. The resulting quaternary ammonium salt is then treated with anhydrous TFA to remove the BOC protecting group, and the resulting amine used for preparation of compound 3.
Procedure 2: A compound 29 containing a tertiary amine in the $R^{2c}R^{2d}N$- or $AA^3$ element is prepared. If histidine is present as the $AA^1$ component, the imidazole ring is protected next by treatment with di-t-butyl-dicarbonate and $Et_3N$ in methanol or DMF. The tertiary amine present in the $AA^3$ or $R^{2c}R^{2d}N$- element is then quaternized by treatment with an alkyl halide in DMF.

Step C7:

Protecting groups are removed from 29 by hydrogenolysis, giving 30.

| | |
|---|---|
| 22 | BOC-Cal[CH(OH)$CH_2$]Val lactone |
| 23 | Cal[CH(OH)$CH_2$]Val lactone |
| 24 | BOC-$AA^1$-Cal[CH(OH)$CH_2$Val lactone |
| 25 | BOC-$AA^2$-$AA^1$-Cal[CH(OH)$CH_2$]Val lactone |
| 26 | $R^{2a}(R^{2b})$X-CO-$AA^2$-$AA^1$-Cal[CH(OH)$CH_2$]Val lactone |
| 27 | $R^{2a}(R^{2b})$X-CO-$AA^2$-$AA^1$-Cal[CH(OTBDMS)$CH_2$]Val-OH |
| 28 | $R^{2a}(R^{2b})$X-CO-$AA^2$-$AA^1$-Cal[CH(OTBDMS)$CH_2$]Val-$AA^3$-$NR^{2c}R^{2d}$ |
| 29 (30) | $R^{2a}(R^{2b})$X-CO-$AA^2$-$AA^1$-Cal[CH(OH)$CH_2$]Val-$AA^3$-$NR^{2c}R^{2d}$ |

Route D:

Step D1:

Lactone 22 is treated with potassium hydroxide to give the corresponding hydroxyacid 31. The hydroxyacid 31 is treated with t-butyldimethylsilyl chloride and imidazole, then with HOAc in THF-$H_2O$, giving the protected hydroxyacid 32.

Step D2:

Protected hydroxyacid 32 is coupled with 3, giving 33. Additional reactive functional groups in the amino component $R^{2c}R^{2d}NH$ are protected with protecting groups such as CBZ for amino and guanidino groups, benzyl ethers for alcohols, and benzyl esters for carboxylic acids.

Step D3:

The BOC protecting group is removed from 33 by treatment with anhydrous TFA, giving 34.

Step D4:

Compound 34 is coupled sequentially with BOC-protected amino acids (BOC-$AA^1$ and Boc-$AA^2$) or with a dipeptide with an N-terminal BOC protecting group (BOC-$AA^1$-$AA^1$), giving coupled product 35.

Step D5:

The N-terminal BOC protecting group of 35 is removed by treatment with anhydrous 'TFA in $CH_2Cl_2$, giving 36.

Step D6:

Compound 36 is treated with an acylating agent represented $R^{2a}(R^{2b})X$-CO-W, as defined above. This gives acylated tripeptide derivative 28. Additional reactive functional groups in $R^{2a}(R^{2b})X$-CO-W are protected with protecting groups as described above.

Step D7:

The silyl protecting group is removed from 28 by treatment with fluoride, giving 29.

Step D8:

In some cases a quaternized amino group is present in the C-terminal $R^{2c}R^{2d}N$- element of compound 30. In this case, one of the following procedures is followed:

Procedure 1: A tertiary amine within $R^{2c}$ or $R^{2d}$ of $R^{2c}R^{2d}NH$ is quaternized by treatment of $R^{2c}R^{2d}N$-BOC with an alkyl halide and $KHCO_3$ in methanol or ethanol or with an alkyl halide in DMF. The resulting quaternary ammonium salt is then treated with anhydrous TFA to remove the BOC protecting group, and the resulting amine used to prepare 3 as described in route A.

Procedure 2: A compound 29 is prepared as described above. If histidine is present as the $AA^1$ component, the imidazole ring is protected next by treatment with di-t-butyldicarbonate and $Et_3N$ in methanol or DMF. The tertiary amine present in the $R^{2c}R^{2d}N$- element is then quaternized by treatment with an alkyl halide in DMF.

Step D9:

It is understood that during the steps above, the reactive functional groups in the side-chains of amino acid components are protected with protecting groups, which are removed in the above hydrogenolysis step. These protecting groups may be CBZ for amines and guanidines, and benzyl ethers for alcohols. If histidine is present as $AA^1$, the side-chain imidazole ring is protected with a BOC group which is removed by TFA in step D3. Thereafter the side-chain of histidine is left unprotected. Alternatively, histidine with a DNP protecting group may be used. In this case, the DNP group is removed after step D10 by treatment of 30 with thiophenol.

Step D10:

Protecting groups are removed from 29 by hydrogenolysis, giving 30.

| | |
|---|---|
| 31 | BOC-Cal[CH(OH)$CH_2$]Val-OH |
| 32 | BOC-Cal[CH(OTBDMS)$CH_2$]Val-OH |
| 33 | BOC-Cal[CH(OTBDMS)$CH_2$]Val-$AA^3$-$NR^{2c}R^{2d}$ |
| 34 | Cal[CH(OTBDMS)$CH_2$]Val-$AA^3$-$NR^{2c}R^2$ |
| 35 | BOC-$AA^2$-$AA^1$-Cal[CH(OTBDMS)$CH_2$]Val-$AA^3$-$NR^{2c}R^{2d}$ |
| 36 | $AA^2$-$AA^1$-Cal[CH(OTBDMS)$CH_2$]Val-$AA^3$-$NR^{2c}R^{2d}$ |

The novel peptides of the present invention possess a high degree of activity in treating renin-associated hypertension, hyperaldosteronism and/or congestive heart failure in humans, as well as in other

warm-blooded animals such as mice, rats, horses, dogs and cats.

For these purposes, the peptides of the present invention may be administered orally, parenterally (including subcutaneous injections, intravenous, intramuscular, intrasternal injection or infusion techniques), by inhalation spray, or rectally, in dosage unit formulations containing conventional non-toxic, pharmaceutically-acceptable carriers, adjuvants and vehicles.

Thus, in accordance with the present invention there is further provided a method of treating and a pharmaceutical composition for treating renin-associated hypertension, hyperaldosteronism, and/or congestive heart failure. This treatment involves administering to a patient in need of such treatment a pharmaceutical composition comprising a pharmaceutical carrier, optionally with an adjuvant, and a therapeutically-effective amount of a peptide of the formula:

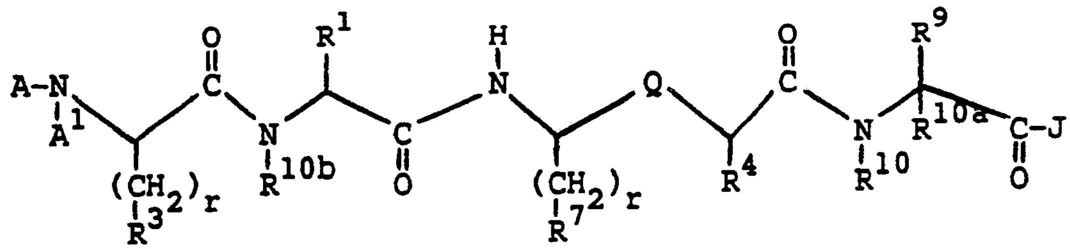

wherein A, $A^1$, $R^1$, $R^3$, $R^4$, $R^7$, $R^9$, $R^{10}$, $R^{10a}$, $R^{10b}$, J and r are defined above, or a pharmaceutically-acceptable salt thereof.

These pharmaceutical compositions may be in the form of orally-administrable suspensions or tablets; nasal sprays; sterile injectable preparations, for example, as sterile injectable aqueous or oleagenous suspensions; or suppositories.

When administered orally as a suspension, these compositions may contain microcrystalline cellulose for imparting bulk, alginic acid or sodium alginate as a suspending agent, methylcellulose as a viscosity enhancer, and sweetners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions may be prepared as solutions in saline, employing benzyl alcohol or other suitable preservatives, absorption promoters to enhance bioavailability, flourocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquify and/or dissolve in the rectal cavity to release the drug.

Dosage levels of the order of 0.02 to 2.0 grams-per-day are useful in the treatment of the above-indicated conditions, with oral doses two-to-five times higher. For example, renin-associated hypertension and hyperaldosteronism are effectively treated by the administration of from 10 to 50 milligrams of the compound per kilogram of body weight from one to three times per day. It will be understood, however, that the specific dose level and frequency of dosage for any particular patient will depend upon a variety of factors including the activity of the specific compound employed, the metabolic stability and length of action of that compound, the age, body weight, general health, sex, diet, mode and time of administration, rate of excretion, drug combination the severity of the particular condition, and the host undergoing therapy.

The present invention is also directed to combinations of the novel renin-inhibitory peptides of Formula I with one or more antihypertensive agents selected from the group consisting of diuretics, α-and/or β-adrenergic blocking agents, CNS-acting agents, adrenergic neuron blocking agents, vasodilators, angiotensin I converting enzyme inhibitors, calcium channel blockers, and other antihypertensive agents.

For example, the compounds of this invention can be given in combination with such compounds or salt or other derivative forms thereof as:

Diuretics:

acetazolamide; amiloride; bendroflumethiazide; benzthiazide; bumetanide; chlorothiazide; chlorthalidone; cyclothiazide; ethacrynic acid; furosemide; hydrochlorothiazide; hydroflumethiazide; indacrinone (racemic

mixture, or as either the (+) or (-) enantiomer alone, or a manipulated ratio, e.g., 9:1 of said enantiomers, respectively); metolazone; methyclothiazide; muzolimine; polythiazide; quinethazone; sodium ethacrynate; sodium nitroprusside; spironolactone; ticrynafen; triamterene; trichlormethiazide;

α-Adrenergic Blocking Agents:

dibenamine; phentolamine; phenoxybenzamine; prazosin; tolazoline;

β-Adrenergic Blocking Agents:

atenolol; metoprolol;

nadolol; propranolol; timolol;

((±)-2-[3-(tert-butylamino)-2-hydroxypropoxy]-2-furananilide) (ancarolol);
(2-acetyl-7-(2-hydroxy-3-isopropylaminopropoxy)benzofuran HCl) (befunolol);
((±)-1-(isopropylamino)-3-(p-(2-cyclopropylmethoxyethyl)-phenoxy)-2-propranol HCl) (betaxolol);
(1-[(3,4-dimethoxyphenethyl)amino]-3-(m-tolyloxy)-2-propanol HCl) (bevantolol);
((±)-1-(4-((2- isopropoxyethoxy)methyl)phenoxy)-3-isopropylamino-2-propanol)fumarate) (bisoprolol);
(4-(2-hydroxy-3-[4-(phenoxymethyl)-piperidino]propoxy)-indole);
(carbazolyl-4-oxy-5,2-(2-methoxyphenoxy)-ethylamino-2-propanol);
(1-((1,1-dimethylethyl)amino)-3-((2-methyl-1H-indol-4-yl)oxy)-2-propanol benzoate) (bopindolol);
(1-(2-exobicyclo[2.2.1]-hept-2-ylphenoxy)-3-[(1-methylethyl)-amino]-2-propanol HCl) (bornaprolol);
(o-[2-hydroxy-3-[(2-indol-3-yl-1,1-dimethylethyl)amino]propoxy]benzonitrile HCl) (bucindolol);
(α-[(tert.butylamino)methyl]-7-ethyl-2-benzofuranmethanol) (bufuralol);
(3-[3 acetyl-4-[3-(tert.butylamino)-2-hydroxypropyl]phenyl]-1,1-diethylurea HCl) (celiprolol);
((±)-2-[2-[3-[(1,1-dimethylethyl)amino]-2-hydroxypropoxy]phenoxy]-N-methylacetamide HCl) (cetamolol);
(2-benzimidazolyl-phenyl(2-isopropylaminopropanol));
((±)-3′-acetyl-4′-(2-hydroxy-3-isopropylaminopropoxy)acetanilide HCl) (diacetolol);
(methyl-4-[2-hydroxy-3-[(1-methylethyl)aminopropoxy]]benzenepropanoate HCl) (esmolol);
(erythro-DL-1-(7 methylindan-4-yloxy)-3-isopropylaminobutan-2-ol);
(1-tert.butylamino)-3-[O-(2-propynyloxy)phenoxy]-2-propanol (pargolol);
(1-(tert.butylamino)-3-[o-(6-hydrazino-3-pyridazinyl)phenoxy]-2-propanol diHCl) (prizidilol);
((-)-2-hydroxy-5-[(R)-1-hydroxy-2-[(R)-(1-methyl-3-phenylpropyl)amino]ethyl]benzamide);
(4-hydroxy-9-[2-hydroxy-3-(isopropylamino)-propoxy]-7-methyl-5H-furo[3,2 g][1]-benzopyran-5-one) (iprocrolol);
((-)-5-(tert.butylamino)-2-hydroxypropoxy]-3,4-dihydro-1-(2H)-naphthalenone HCl) (levobunolol);
(4-(2-hydroxy-3-isopropylamino-propoxy)-1,2-benzisothiazole HCl);
(4-[3-(tert.butylamino)-2-hydroxypropoxy]-N-methylisocarbostyril HCl);
((±)-N-2-[4-(2-hydroxy-3-isopropyl aminopropoxy)phenyl]ethyl-N′-isopropylurea) (pafenolol);
(3-[[(2-trifluoroacetamido)ethyl]amino]-1-phenoxypropan-2-ol);
(N-(3-(o-chlorophenoxy)-2-hydroxypropyl)-N′-(4′-chloro-2,3-dihydro-3-oxo-5-pyridazinyl)ethylenediamine);
((±)-N-[3-acetyl-4-[2-hydroxy-3-[(1-methylethyl)amino]propoxy]phenyl]butanamide) (acebutolol);
((±)-4′-[3-(tert-butylamino)-2-hydroxypropoxy]spiro[cyclohexane-1,2′-indan]-1′-one) (spirendolol);
(7-[3-[[2-hydroxy-3-[(2-methylindol-4-yl)oxy]propyl]amino]butyl]thiophylline) (teoprolol);
((±)-1-tert.butylamino-3-(thiochroman-8-yloxy)-2-propanol) (tertatolol);
((±)-1-tert.butylamino-3-(2,3-xylyloxy)-2-propanol HCl) (xibenolol);
(8-[3-(tert.butylamino)-2-hydroxypropoxy]-5-methylcoumarin) (bucumolol);
(2-(3-(tert.butylamino)-2-hydroxy-propoxy)benzonitrile HCl) (bunitrolol);
((±)-2′-[3-(tert-butylamino)-2-hydroxypropoxy-5′-fluorobutyrophenone) (butofilolol);
(1-(carbazol-4-yloxy)-3-(isopropylamino)-2-propanol) (carazolol);
(5-(3-tert.butylamino-2-hydroxy)propoxy-3,4-dihydrocarbostyril HCl) (carteolol);
(1-(tert.butylamino)-3-(2,5-dichlorophenoxy)-2-propanol) (cloranolol);
(1-(inden-4(or 7)-yloxy)-3-(isopropylamino)-2-propanol HCl) (indenolol);
(1-isopropylamino-3-[(2-methylindol-4-yl)oxy]-2-propanol) (mepindolol);
(1-(4-acetoxy-2,3,5-trimethylphenoxy)-3-isopropylaminopropan-2-ol) (metipranolol);
(1-(isopropylamino)-3-(o-methoxyphenoxy)-3-[(1-methylethyl)amino]-2-propanol) (moprolol);
((1-tert.butylamino)-3-[(5,6,7,8-tetrahydro-cis-6,7-dihydroxy-1-naphthyl)oxy]-2-propanol) (nadolol);
((S)-1-(2-cyclopentylphenoxy)-3-[(1,1-dimethylethyl)amino]-2-propanol sulfate (2:1)) (penbutolol);
(4′-[1-hydroxy-2-(amino)ethyl]methanesulfonanilide) (sotalol);
(2-methyl-3-[4-(2-hydroxy-3-tert.butylaminopropoxy)phenyl]-7-methoxy-isoquinolin-1-(2H)-one);
(1-(4-(2-(4-fluorophenyloxy)ethoxy)phenoxy)-3-isopropylamino-2-propanol HCl);
((-)-p-[3-[(3,4-dimethoxyphenethyl)amino]-2-hydroxy propoxy]-8-methylcinnamonitrile) (pacrinolol);
((±)-2-(3′-tert.butylamino-2-hydroxypropylthio)-4-(5′-carbamoyl-2-thienyl)thiazole HCl) (arotinolol);

((±)-1-[p-[2-(cyclopropylmethoxy)ethoxy]phenoxy]-3-(isopropylamino)-2-propanol) (cicloprolol);
((±)-1-[(3-chloro-2-methylindol-4-yl)oxy]-3-[(2-phenoxyethyl)amino]-2-propanol) (indopanolol);
((±)-6-[[2-[[3-(p-butoxyphenoxy)-2-hydroxypropyl]amino]ethyl]amino]-1,3-dimethyluracil) (pirepolol);
(4-(cyclohexylamino)-1-(1-naphtholenyloxy)-2-butanol);
(1-phenyl-3-[2-[3-(2-cyanophenoxy)-2-hydroxypropyl]aminoethyl]hydantoin HCl);
(3,4-dihydro-8-(2-hydroxy-3-isopropylaminopropoxy)-3-nitroxy-2H-1-benzopyran) (nipradolol);
α- and β-Adrenergic Blocking Agents:
((±)-1-tert-butylamino)-3-[o-[2-(3-methyl-5-isoxazolyl)vinyl]phenoxy]-2-propanol) (isoxaprolol);
(1-isopropylamino-3-(4-(2-nitroxyethoxy)phenoxy)-2-propanol-HCl);
(4-hydroxy-α-[[3-(4-methoxyphenyl)-1-methylpropyl]aminomethyl]-3-(methylsulfinyl)-benzmethanol HCl) (sulfinalol);
(5-[1-hydroxy-2-[[2-(o-methoxyphenoxy)ethyl]amino]ethyl]-2-methylbenzenesulfonamide HCl);
(5-[1-hydroxy-2-[(1-methyl-3-phenylpropyl)amino]ethyl]salicylamide HCl) (labetalol);
(1-((3-chloro-2-methyl-1H-indol-4-yl)oxy)-3-((2-phenoxyethyl)amino)-2-propanol-hydrogenmalonate) (ifendolol);
(4-(2-hydroxy-3-[(1-methyl-3-phenylpropyl)amino]propoxy)benzeneacetamide);
(1-[3-[[3-(1-naphthoxy)-2-hydroxypropyl]-amino]-3,3-dimethyl-propyl]-2-benzimidazolinone);
(3-(1-(2-hydroxy-2-(4-chlorophenylethyl)-4-piperidyl)3,4-dihydroxy)quinoxolin-2(1H)-one);
CNS-Acting Agents:
clonidine; methyldopa;
Adrenergic Neuron Blocking Agents:
guanethidine; reserpine and other rauwolfia alkaloids such as rescinnamine;
Vasodilators:
diazoxide; hydralazine; minoxidil;
Angiotensin I Converting Enzyme Inhibitors:
1-(3-mercapto-2-methyl-1-oxopropyl)-L-proline (captopril);
(1-(4-ethoxycarbonyl-2,4(R,R)-dimethyltutanoyl)indoline-2(S)-carboxylic acid);
(2-[2-[[1-(ethoxycarbonyl)-3-phenyl-propyl]amino]-1-oxopropyl]-1,2,3,4-tetrahydro-3-isoquinoline carboxylic acid);
((S)-1-[2-[[1-(ethoxycarbonyl)-3-phenylpropyl]amino]-1-oxopropyl]octahydro-1H-indole-2-carboxylic acid HCl);
(N-cyclopentyl-N-(3-(2,2-dimethyl-1-oxopropyl)thiol-2-methyl-1-oxopropyl)glycine) (pivalopril);
((2R,4R)-2-(2-hydroxyphenyl)-3-(3-mercaptopropionyl)-4-thiazolidinecarboxylic acid);
(1-(N-[1(S)-ethoxycarbonyl-3-phenylpropyl]-(S)-alanyl)cis,syn-octahydroindol-2(S)-carboxylic acid HCl);
((-)-(S)-1-[(S)-3-mercapto-2-methyl-1-oxopropyl]indoline-2-carboxylic acid);
([1(S),4S]-1-[3-(benzoylthio)-2-methyl-1-oxopropyl]-4-phenylthio-L-proline;
(3-([1-ethoxycarbonyl-3-phenyl-(1S)-propyl]amino)2,3,4,5-tetrahydro-2-oxo-1-(3S)-benzazepine-1-acetic acid HCl);
(N-(2-benzyl-3-mercaptopropanoyl)-S-ethyl-L-cysteine) and the S-methyl analogue;
(N-(1(S)-ethoxycarbonyl-3-phenylpropyl)-L-alanyl-L-proline maleate) (enalapril);
N-[1-(S)-carboxy-3-phenylpropyl]-L-alanyl-1-proline;
$N^2$-[1-(S)-carboxy-3-phenylpropyl]-L-lysyl-L-proline (lisinopril);
Calcium Channel Blockers:
α-[3-[[2-(3,4- dimethoxyphenyl)ethyl]methylamino]propyl]-3,4-dimethoxy-α-(1-methylethyl)-benzeneacetonitrile (verapamil);
1,4-dihydro-2,6-dimethyl-4-(2-nitrophenyl)-3,5-pyridinedicarboxylic acid dimethyl ester (nifedipine);
2-(2,2-dicyclohexylethyl)piperidine (perhexiline);
N-(1-methyl-2-phenylethyl)- -phenylbenzenepropanamine (prenylamine);
3-(aminosulfonyl)-4-chloro-N-(2,3-dihydro-2-methyl-1H-indol-1-yl)benzamide (indapamide);
(2′-(2-diethylaminoethoxy)-3-phenylpropiophenone (etafenone);
(4-[4,4-bis-(4-fluorophenyl)butyl]-N-(2,6-dimethylphenyl)-1-piperazineacetamide) (lidoflazine);
(2-(N-benzyl-N-methylamino)ethylmethyl-2,6-dimethyl-4-(m-nitrophenyl)-1,4-dihydro-3,5-pyridinedicarboxylate HCl) (nicardipine);
(N-(3,4-dimethoxyphenethyl)-2-(3,4-dimethoxyphenyl)-N-methyl-m-dithiane-2-propylamine-1,1,3,3-tetraoxide) (tiapamil);
(5,6-dimethoxy-2-(3-[(α-(3,4-dimethoxy)phenylethyl)methylamino]propyl)phthalimidine) (falipamil);
(β-[(2-methylpropoxy)methyl]-N-phenyl-N-phenylmethyl-1-pyrrolidineethanamine HCl monohydrate) (bepridil);

((+)-cis-3-(acetyloxy)-5-[2-(dimethylamino)ethyl]-2,3-dihydro-2-(4-methoxyphenyl)-1,5-benzothiazepin-4-(5H)-one) (diltiazem);
((E)-1-[bis-(p-fluorophenyl)methyl]-4-cinnamylpiperazine di HCl) (flunarizine);
(5-[(3,4-dimethoxyphenethyl)methylamino]-2-isopropyl-2-(3,4,5-trimethoxyphenyl)valeronitrile (gallopamil);
(ethylmethyl(2,3- dichlorophenyl)-1,4-dihydro-2,6-dimethyl-3,5-pyridinedicarboxylate (felodipine);
(isopropyl-2-methoxyethyl-1,4-dihydro-2,6-dimethyl-4-(3-nitrophenyl)-3,5-pyridinecarboxylate) (nimodipine);
(3-ethyl-5-methyl-1,4-dihydro-2,6-dimethyl-4-(3-nitrophenyl)-3,5-pyridine-dicarboxylate) (nitrendipine);
Other Antihypertensive Agents:
aminophylline; cryptenamine acetates and tannates; deserpidine; meremethoxylline procaine; pargyline; trimethaphan camsylate; and the like, as well as admixtures and combinations thereof.

Typically, the individual daily dosages for these combinations can range from about one fifth of the minimally-recommended clinical dosages to the maximum recommended levels for the entities when they are given alone. Coadministration is most readily accomplished by combining the active ingredients into a suitable unit dosage form containing the proper dosages of each. Other methods of coadministration are, of course, possible.

The renin-inhibitory novel peptides of the present invention may also be utilized in in vivo or in vitro diagnostic methods for the purpose of establishing the significance of renin as a causative or contributory factor in hypertension, hyperaldosteronism or congestive heart failure in a particular patient.

In the in vivo method, a novel peptide of the present invention is administered to a patient, preferably by intravenous injection, although parenteral administration is also suitable, at a hypotensive dosage level in a single dose of from 0.1 to 10 mg per kg of body weight, and the resulting transitory fall in blood pressure, if it occurs, indicates supranormal plasma renin levels.

In vitro methods which may be employed involve incubating a body fluid, preferably plasma, with a novel peptide of the present invention according to methods described in Boger et al,, J. Med. Chem., 1985, 28, 1789-1790.

The following are intended to exemplify the present invention, without, however, limiting it.

## EXAMPLE 1

### Preparation of Boc-Phe-His-ACHPA-Lys-NHCH$_2$(pyridin-4-yl) hydrochloride [N$^{\alpha}$-t-butoxycarbonyl-L-Phenylalanyl-L-histidyl-(3S,4S)-4-amino-5-cyclohexyl-3-hydroxypentanoyl-L-lysyl-4-(aminomethyl)pyridine amide hydrochloride]

#### Step A: Boc-Lys(Cbz)-NHCH$_2$(pyridin-4-yl)

To a cooled (-15°C), stirring solution of N$^{\alpha}$-Boc-N$^{\epsilon}$-Cbz-Lys-OH [Boc-Lys(Cbz)-OH] (3.8 g, 10 mmoles) and N methylmorpholine (1.10 ml, 10 mmoles) in dry THF (50 ml), isobutylchloroformate (1.29 ml, 10 mmoles) was added in four portions. After 5 min., 4-(aminomethyl)pyridine (1.12 ml, 11 mmoles) was added, and the mixture was stirred for 2 hours at -15°C and then at room temperature for 4 hours. A saturated solution of $NaHCO_3$ (50 ml) was added, and the mixture was stirred for 30 min., before the organic phase was separated and the aqueous phase was extracted with ethylacetate (50 ml).

The combined organic layer was washed with water, dried ($MgSO_4$),and concentrated in vacuo to give the product as a foam, which upon trituration with ether and filtration gave the pure product as a white solid (4.1 g, 88%), m.p. 109-110°C. TLC (silica gel) (EtOAc): $R_f$= 0.29 . NMR ($CDCl_3$): consistent with the structure. FAB mass spectral data: 471 (M+H), 415 (M-$C_4H_8$+H), 381 (M-Boc+H)

#### Step B: ACHPA-OEt hydrochloride

A cooled (ice-bath) solution of Boc-ACHPA-OEt (65 g) in absolute ethanol (400 ml) was stirred vigorously, as anhydrous HCl was bubbled in. After 10 minutes, the cooling bath was removed and the addition of HCl was continued until the temperature of the solution had reached 35°C. At this stage, TLC showed complete disappearance of the starting material.

The solution was concentrated to a thick oil, which was reconcentrated twice from toluene (2X100 ml)

and dried in vacuo overnight (55.5 g). TLC (silica gel) in $CH_2Cl_2$-MeOH-AcOH (90:10:1) $R_f$ 0.4.

Step C: Boc-Phe-His(Boc)-ACHPA-OEt

To a cooled (0 °C) solution of ACHPA-OEt hydrochloride (55.5 g) in $CH_2Cl_2$ (500 ml) were added diisopropylethylamine (6 ml) and then Boc-Phe-His(Boc)-OH (96.5 g), giving a clear solution. 1-Hydroxybenzotriazole hydrate (34 g) and additional diisopropylethylamine (35 ml) were then added, followed by DCC (40 g). The mixture was stirred (1 hour) and the ice bath was removed, with stirring being continued (18 hours).

The dicyclohexylurea (DCU) formed was removed by filtration and the filtrate was concentrated to a thick slurry, which was taken up in ethylacetate (6 liters) and 0.5M citric acid solution (2 liters). The organic phase was separated and washed with citric acid solution, water, saturated $NaHCO_3$ (600 ml), water (2X2 liters) and brine, dried ($Na_2SO_4$). The resulting solution was concentrated to a thick slurry.

The pure product was obtained as a white solid (97 g), after crystallization from ethylacetate. m.p. 156.7-158.8 °C. TLC in ethylacetate-hexane (4:1) Rf 0.35. [α]D -23.6

Step D: Boc-Phe-His-ACHPA-$NHNH_2$

410g of hydrazine hydrate was added to a solution of Boc-Phe-His(Boc)-ACHPA-OEt (97 g) in methanol (1.5 liters) at 0 °C, and the reaction was allowed to warm up to room temperature over a period of 1 hour, before being cooled again (ice-bath) and stirred for 18 hours. The mixture was concentrated to a thick slurry, then diluted with water (500 ml) and filtered. The solid was washed with water (3X500 ml) on the filter and dissolved in boiling 95% ethanol (700 ml), filtered while hot, and cooled.

The resulting solid was filtered and dried to give the product (74.5 g), m.p. 186.1-187.7 °C. TLC (silica gel) in $CH_2Cl_2$-MeOH (3:1): Rf 0.5. [α]D -37.5.

Step E: Boc-Phe-His-ACHPA-Lys(Cbz)-$NHCH_2$(pyridin-4-yl)

Boc-Lys(Cbz)-$NHCH_2$(pyridin-4-yl) (0.6g, 1.28 mmole) was dissolved in $CH_2Cl_2$ (2 ml) and anhydrous TFA (2 ml) was added. The mixture was stirred at room temperature for 40 min and concentrated under reduced pressure to give the corresponding Lys(Cbz)$NHCH_2$(pyridin-4-yl) bistrifluoroacetate salt as a semisolid mass, which was used directly in the coupling step described below.

A solution of Boc-Phe-His-ACHPA-$NHNH_2$ (0.66g, 1.06 mmols) in dry DMF (8 ml) was cooled to -30 °C under $N_2$ and conc. HCl (12 N) (0.54 ml, 6.36 mmols) was added (pH ~ 1.0) followed by the addition of isoamylnitrite (0.20 ml, 1.48 mmols). The reaction mixture (positive to KI-starch paper) was stirred at -18 °C to -22 °C for 3-4 hours and formation of the acyl azide was monitored on TLC using $CHCl_3$:MeOH:$NH_4OH$ (40:10:1). At the completion of the azide formation, a pre-cooled (-20 °C) solution of Lys(Cbz)$NHCH_2$-(pyridin-4-yl)•2TFA and diisopropylethylamine (1.65 ml, 9.5 mmols) in DMF (5 ml) was added.

The mixture (pH ~ 10) was stirred at -20 °C for one hour and then stored in the freezer for 48 hours, at which time, the pH of the reaction mixture was found to have dropped to about 9.0. DMF was removed in vacuo and the oily residue was treated with cold water, extracted with ethylacetate containing 10% MeOH) (100 ml) and the organic phase was washed with water, dried ($MgSO_4$) and removed under reduced pressure to give a foam (1.2 g). The crude product was dissolved in warm ethyl acetate (20 ml) and allowed to stand at room temperature overnight.

The product obtained as white gel was filtered, washed with cold ethylacetate (3 ml), and dried to give the an amorphous solid (0.84 g, 82%). HPLC: Purity > 99% ($C_{18}$-Zorbax analytical column using $CH_3CN$: 0.01M $KH_2PO_4$ = 65:35; retention time 5.89 min); NMR ($CD_3OD$): consistent with the desired structure; FAB-(+) mass spec: 952(M+H), 852 (M-Boc+H)

In an alternative procedure, Boc-Phe-His-ACHPA-Lys(Cbz)-$NHCH_2$(pyridin-4-yl) was prepared by coupling Boc-Phe-His(Boc)-OH with the free amine of ACHPA-Lys(Cbz)-$NHCH_2$(pyridin-4-yl), as described below:

Boc-ACHPA-Lys(Cbz)-$NHCH_2$(pyridin-4-yl)

Boc-ACHPA-OH (3.1 g, 10 mmols), Lys(Cbz)-$NHCH_2$(pyridin-4-yl) dihydrochloride (obtained from the treatment of 4.75 g of Boc-Lys(Cbz)-$NHCH_2$(pyridin-4-yl) with HCl in dioxane (4N) (4 ml) at room temperature for one hour), and diisopropylethylamine (6.85 ml) were dissolved in methylene chloride (60 ml), then cooled (0° C), and BOP reagent (6.45g, 14.6 mmols) was added. The mixture was stirred at that temperature 2 hours and then for 24 hours at room temperature. The solvent was removed in vacuo and the residue was dissolved in ethylacetate (100 ml). The organic phase was washed with water, saturated $NaHCO_3$ and water, then dried ($MgSO_4$) and evaporated in vacuo to give an oil, which was purified by medium pressure chromatography (MPLC) on silica gel 60 using 1% MeOH in ethylacetate.

The pure product, Boc-ACHPA-Lys(Cbz)-$NHCH_2$(pyridin 4-yl), was obtained as a foam (4.56 g, 68 %). NMR ($CDCl_3$): consistent with the desired structure; FAB mass spectral data : 668 (M+H), 612 (M-$C_4H_8$ + H) and 568(M-Boc + H).

Boc-Phe-His-ACHPA-Lys(Cbz)-$NHCH_2$(pyridin-4-yl)

Boc-ACHPA-Lys(Cbz)-$NHCH_2$(pyridin-4-yl) (0.67 g, 1 mmol) was dissolved in a solution of HCl in dioxane (4N) (5 ml), and the mixture was stirred at room temperature for 1 hour. Removal of the solvent in vacuo gave ACHPA-Lys(Cbz)-$NHCH_2$(pyridin-4 yl) dihydrochloride as a foam, which was dried in vacuo overnight. The salt was suspended in methylene chloride (15 ml), cooled (0° C) and neutralized with diisopropylethylamine (0.37 ml, 2.2 mmol). Boc-Phe-His(Boc)-OH (0.54 g, 1.1 mmol) and HOBT (0.22 g, 1.6 mmol) were then added, followed by DCC (0.23 g, 1.1 mmol), and the mixture was stirred at 0° C, for four hours and overnight at room temperature.

Dicyclohexylurea (DCU) was filtered off and the filtrate was concentrated in vacuo. The residue was dissolved in ethylacetate (30 ml), and washed with water, saturated $NaHCO_3$, 5% citric acid, and water, then dried ($MgSO_4$) and evaporated in vacuo giving the product as a foam. In order to remove the imidazole protecting group (Boc), the crude product was dissolved in methanol (10 ml) and the solution was saturated with anhydrous ammonia.

After stirring the mixture at room temperature for five hours, the solvent was removed in vacuo to give the crude Boc-Phe-His-ACHPA-Lys(Cbz)$NHCH_2$(pyridin-4-yl) as a foam (0.75 g). The crude material was finally purified by MPLC on silica-gel using ethylacetate: methanol (9:1) giving the pure product as white amorphous solid (0.65 g).

Step F: Boc-Phe-His-ACHPA-Lys-$NHCH_2$(pyridin-4-yl) hydrochloride

A solution of Boc-Phe-His-ACHPA-Lys(Cbz)-$NHCH_2$(pyridin-4-yl) (0.53 g, 0.557 mmol) in methanol (30 ml) was hydrogenated in presence of conc. HCl (0.045 ml) and Pd-C (10%)(0.06 g) at 40 psi and room temperature for one hour. The catalyst was filtered off and the filtrate was evaporated to give the product as glass-like solid (0.5 g), which was dissolved in dry methanol (2 ml) and precipitated with the addition dry ether.

The solid was filtered washed with ether and dried to give a white solid (0.42 g). NMR ($CD_3OD$): consistent with the desired structure. FAB mass spectral data: 818 (M+H), 718 (M-Boc+H). $IC_{50}$ =38nM

## EXAMPLE 2

Preparation of Boc-Phe-His-ACHPA-Lys-$NHCH_2$(piperidin-4-yl) dihydrochloride salt $N^{\alpha}$-t-butoxycarbonyl-L-phenylalanyl-L-histidyl-(3S,4S)-4-amino-5-cyclohexyl-3-hydroxy-pentanoyl-L-lysyl-4-(aminomethyl)-piperidinamide dihydrochloride]

Boc-Phe-His-ACHPA-Lys(Cbz)-$NHCH_2$(pyridin-4-yl) (0.3 g, 0.32 mmol) was dissolved in methanol (20 ml) to which conc. HCl (0.06 ml, 0.64 mmol) and $Pd(OH)_2$ (0.1 g) had been added, and the mixture was hydrogenated for 24 hours at 40 psi and room temperature. The catalyst was filtered off and the filterate was evaporated in vacuo to give the product as a glass-like solid. The solid was dissolved in dry methanol (2 ml) and precipitated with the addition of dry ether. The precipitate was filtered, and dried in vacuo to give the product as white solid (0.22 g). NMR ($CD_3OD$): consistent with the desired structure; FAB mass spectral

data: 824 (M + H), 724 (M-Boc + H). $IC_{50}$ = 338nM

## EXAMPLE 3

Preparation of Boc-Phe-His-ACHPA-Lys-$NHCH_2$(pyridin-2-yl) hydrochloride [$N^{\alpha}$-t-butoxycarbonyl-L-phenylalanyl-L-histidyl-(3S,4S)-4-amino-5-cyclohexyl-3-hydroxy-pentanoyl-L-lysyl-2-(aminomethyl)pyridine amide hydrochloride]

Step A: Boc-Lys(Cbz)-$NHCH_2$(Pyridine-2-yl)

The title compound was prepared in 88% yield according to the procedure in Step A of Example 1, with 2-(aminomethyl)pyridine replacing 4-(aminomethyl) pyridine. mp. 89-90 °C. NMR ($CDCl_3$): consistent with the desired structure; FAB mass spectral data : 471 (M + H), 371 (M-Boc + H).

Step B: Boc-Phe-His-ACHPA-Lys-$NHCH_2$(pyridin-2-yl) hydrochloride

The title compound was prepared according to the method of Example 1, with Lys-$NHCH_2$(pyridin-2-yl) replacing Lys-$NHCH_2$(pyridin-4-yl). NMR ($CD_3OD$): consistent with the desired structure; FAB mass spectral data : 818(M + H), 718(M-Boc + H)

## EXAMPLE 4

Preparation of Boc-Phe-His-ACHPA-Lys-$N(CH_3)CH_2$-(pyridin-4-yl) acetate [$N^{\alpha}$-t-butoxycarbonyl-L-phenylalanyl-L-histidyl-(3S,4S)-4-amino-5-cyclohexyl-3-hydroxy-pentanoyl-L-lysyl-4-(1-methylamino methyl)-pyridinamide acetate]

Step A: $CH_3$-$NHCH_2$(pyridin-4-yl)

To the 50% aqueous, stirring solution of methylamine (170 ml) was added 4-picolylchloride hydrochloride (25 g) at 0 °C in small portions, over a period of 30 min. The mixture was stirred at that temperature for an additional 30 min, then at room temperature for 2 hours and at 60 °C for 1 hour. The reaction was cooled (0 °C), carefully saturated with solid KOH and extracted with ether. The ether layer was dried (anhyd. $NaSO_4$) and concentrated by distillation, and the dark colored liquid was purified by distillation under reduced pressure. b.p. 132-135 °C (at 18 lmm of Hg). NMR($CDCl_3$): 2.43 s(N-$CH_3$),3.75 s($CH_2$),7.22 d-(aromatic),8.52 d(aromatic).

Step B: Boc-Lys(Cbz)-$N(CH_3)CH_2$(pyridin-4-yl)

A solution of Boc-Lys(Cbz)-OH (0.76 g, 2 mmols) in dry THF (8 ml) was cooled in an ice bath and carbonyldiimidazole (0.357 g, 2.2 mmol) was added. The resulting solution was stirred at that temperature for 30 min and for an additional 30 min at room temperature. A solution of $CH_3NHCH_2$-(pyridin-4-yl) (0.27 g, 2.2 mmols) in dry THF (4 ml) was then added and stirred at room temperature for 15 hours.

The resulting mixture was stirred with sat. $NaHCO_3$ (10 ml) for 30 min, extracted with ethyl acetate (60 ml), and the organic phase was washed with water, then dried ($MgSO_4$) and concentrated in vacuo to give a thick oil (1.1 g).

The product was purified by MPLC on silica using ethylacetate: hexane (4:1). Yield 0.9 g (thick colorless oil). TLC: Rf 0.31 in ethylacetate-hexane (4:1); NMR ($CDCl_3$): consistent with the desired structure; FAB mass spectral data: 485 (M + H), 385 (M-Boc + H)

Step C: Boc-Phe-His-ACHPA-Lys(Cbz)-N($CH_3$)$CH_2$(pyridin-4-yl)

The title compound was prepared according to the method of Example 1, with Lys(CbZ)-N($CH_3$)$CH_2$-(pyridin-4-yl) replacing Lys(Cbz)-NH$CH_2$(pyridin-4-yl) as the amine part in step E. The crude product was purified by MPLC on silica-gel using ethylacetate-methanol(8:1.5). TLC: Rf 0.23 in ethylacetate-methanol-(8:1). NMR($CD_3OD$): consistent with the desired structure; FAB mass spectral data: 966 (M+H), 866 (M-Boc+H).

Step D: Boc-Phe-His-ACHPA-Lys-N($CH_3$)$CH_2$(pyridin-4-yl) acetate

To a solution of Boc-Phe-His-ACHPA-Lys-N($CH_3$)$CH_2$(pyridin-4-yl) (0.15 g, 0.155 mmol) in methanol (15 ml) was added AcOH (0.012 ml) followed by Pd-C(10%)(0.05 g). The mixture was stirred under an atmosphere of hydrogen overnight (18 hours). TLC at the end of this period showed complete reaction. The catalyst was filtered off and the filtrate was concentrated to dryness in vacuo, giving a semi-soild mass, which was triturated with dry ether to give white powder.

The solid was filtered and dried (0.12 g). HPLC: 98.9% pure; $C_{18}$ Dynamax column, MeOH:0.1% TFA-$H_2O$ (50:50); retention time 5.3 min.

NMR($CD_3OD$): consistent with the structure. FAB mass spectral data: 832(M+H), 732(M-Boc+H).

## EXAMPLE 5

Preparation of Boc-Phe-His-ACHPA-Lys-N($CH_3$)$CH_2CH_2$-(pyridin-2-yl) acetate [$N^{\alpha}$-t-butoxycarbonyl-L-phenylalanyl-L-histidyl-(3S,4S)-4-amino-5-cyclohexyl-3-hydroxypentanoyl-L-lysyl-2-(2-methylamino ethyl)-pyridinamide acetate]

The title compound was prepared according to the procedure described in Example 4, with $CH_3NHCH_2CH_2$ (pyridin-2-yl) replacing $CH_3NHCH_2$-(pyridin-4-yl). NMR ($CD_3OD$): consistent with the desired structure; FAB mass spectral data: 846 (M+H). $IC_{50}$=225nM.

## EXAMPLE 6

Preparation of Boc-Tyr(OMe)-His-ACHPA-Lys-NH$CH_2$-(pyridin-4-yl)[$N^{\alpha}$-t-butoxycarbonyl-O-methyl-L-tyrosyl-L-histidyl-(3S,4S)-4-amino-5-cyclohexyl-3-hydroxy-pentanoyl-L-lysyl-4-(aminomethyl)pyridine amide]

Step A: Boc-Tyr(OMe)-N-hydroxysuccinimide ester

To a solution of N hydroxysuccinimide (4.21 g, 35.6 mmols) in dioxane (210 ml) was added a solution of Boc-O-methyl-L-tyrosine (10 g, 33.8 mmols), and the mixture was cooled to 0°C. DCC (7.6 g, 36.8 mmols) was added and the mixture was stirred at 0°C for 6 hours and at room temperature overnight. DCU was filtered off, and the filtrate was evaporated in vacuo. The residue was dissolved in acetone (20 ml), cooled and filtered to remove residual DCU. The filtrate was concentrated to dryness, and the residue was triturated with hexane and filtered to give the product as white crystalline solid (14.25 g). mp. 147.7-148.9°C; NMR ($CDCl_3$): consistent with the structure.

Step B: Boc-His(DNP)-ACHPA-Lys(Cbz)-NH$CH_2$(pyridin-4-yl)

A solution of Boc-ACHPA-Lys(Cbz)-NH$CH_2$-(pyridin-4-yl)(from step E of Example 1) in MeOH saturated with HCl was stirred 1 hour at room temperature whereupon it was thoroughly evaporated in vacuo. This material (0.0039mol) was combined in methylene chloride (20 ml) with diisopropylethylamine (2.74 ml, 4

equiv.) and Boc(N-im DNP)-Histidine (2.17 gm, 1.3 gm) and benzotriazol-1-yloxytris (dimethyl-amino)-phosphoniumhexafluorophosphate ("BOP") 2.28 gm. After stirring for 14 hours, this reaction was evaporated in vacuo, redissolved in EtOAc, washed with HCl(IN), sat. $NaHCO_3$ dried over $Na_2SO_4$, evaporated and chromatographed (silica, 9:1:1 EtOAc:acetonitrile:MeOH) to give 2.3 gm of the title compound. TLC (silica, 9:1:1 EtOAc:acetonitrile: MeOH) $R_f = 0.50$. HPLC(RP-18,6:4 0.1% phosphate buffer:acetonitrile) rt = 14.35 min. NMR consistent with structure. FAB mass spectral data: 731(M + H).

Step C: Boc-Tyr(OMe)-His(DNP)-ACHPA-Lys(Cbz)-NHCH$_2$-(pyridin-4-yl)

Boc-His(DNP)-ACHPA-Lys(Cbz)-NHCH$_2$(pyridin-4-yl) (0.82 g, 0.85 mmol) was dissolved in methanol saturated with anhydrous HCl (4 ml) and stirred at room temperature for 1 hour 30 min. The solvent was removed in vacuo and the yellow foam was dried overnight in a desicator over $P_2O_5$. The foam was dissolved in dry DMF (6 ml) and neutralized by addition of diisopropylethylamine (0.37 ml, 2.1 mmol) at 0 °C. Boc-Tyr(OMe)-N-hydroxysuccinimide ester (0.34 g, 0.87 mmol) was added to the above solution and stirred at room temperature for 48 hours. DMF was removed in vacuo and the residue was dissolved in ethylacetate. The organic phase was washed with water, dried ($MgSO_4$) and concentrated to dryness in vacuo to give the crude product as a foam. The material was purified by MPLC on silica gel using ethylacetate-methanol (15:1). Yield: 0.85 g (yellow foam; 87%). NMR($CD_3OD$): consistent with the desired structure; FAB mass spectral data: 1149(M + H)

Step D: Boc-Tyr(OMe)-His-ACHPA-Lys(Cbz)-NHCH$_2$-(pyridin-4-yl)

The fully protected peptide (0.8 g) was dissolved in methylene chloride (5 ml) and treated with thiophenol (0.58 ml). The mixture was stirred at room temperature for 4 hours and then concentrated in vacuo. The residue was triturated with ether and filtered. The crude product was purified by MPLC on LH-20 using methanol. The product, thus obtained, was repurified by flash chromatography on silica-gel using ethylacetate-methanol (9:1) to give the pure product as a foam (0.56 g). TLC (silica gel): Rf 0.64 in chloroform-methanol-$NH_4OH$ (40:10:1); Rf 0.40 in ethylacetate-methanol (9:1). NMR ($CD_3OD$): consistent with the assigned structure; FAB mass spectral data: 983(M + H) and 883(M-Boc + H).

Step E: Boc-Tyr(OMe)-His-ACHPA-Lys-NHCH$_2$(pyridin-4-yl)

Boc-Tyr(OMe)-His-ACHPA-Lys(Cbz)-NHCH$_2$-(pyridin-4-yl) (0.48 g, 0.489 mmol) was dissolved in methanol (7 ml), and conc. HCl (0.085 ml, 1.03 mmol) and $Pd(OH)_2/C$(20%)(0.153 g) were added. The mixture was stirred under $H_2$ atmosphere (at atmospheric pressure) for 5 hours and filtered. The filtrate was concentrated to dryness and the residue was purified first on LH-20 using methanol and then on silica-gel using $CHCl_3$-$CH_3OH$-$NH_4OH$ (40:10:1) giving the pure product as cream colored solid (0.268 g, 65%). HPLC (on Dynamax Scout-$C_{18}$ column): 98.5 % pure; retention time 2.39 min; solvent system $CH_3OH$-0.1% TFA (70:30). NMR($CD_3OD$): consistent with the desired structure; FAB mass spectral data: 848(M + H), 748-(M-Boc + H). $IC_{50}$ = 42nM

## EXAMPLE 7

Preparation of Boc-Phe-His-ACHPA-D-Lys-NHCH$_2$(pyridin-4-yl) hydrochloride [N$^{\alpha}$-t-butoxycarbonyl-L-phenylalanyl-L-histidyl-(3S,4S)-4-amino-5-cyclohexyl-3-hydroxypentanoyl-D-lysyl-4-(aminomethyl)-pyridinamide hydrochloride]

The title compound was prepared according to the procedure described in Example 1, using the appropriate D-lysine derivative. NMR ($CD_3OD$): consistent with the desired structure; FAB mass spectral data: 818(M + H), 718(M-Boc + H).

EXAMPLE 8

Preparation of Boc-Phe-His-ACHPA-Lys-NH(3-quiniclidinyl) dihydrochloride [$N^{\alpha}$-t-butoxycarbonyl-L-phenylalanyl-L-histidyl-(3S,4S)-4-amino-5-cyclohexyl-3-hydroxypentanoyl-L-lysyl-3(R,S)-aminoquiniclidine amide dihydrochloride]

Step A: Boc-Lys-(Cbz)-NH(3-quiniclidin-3-yl)

The title compound was prepared according to the method described in step A of Example 1, with 3-(R,S)-aminoquiniclidine replacing 4-(aminomethyl) pyridine. The product was obtained as a foam in 76% yield. NMR($CD_3OD$): consistent with the desired structure; FAB mass spectral data: 489(M+H)

Step B: Boc-Phe-His-ACHPA-Lys-NH(3-quiniclidinyl) dihydrochloride

The title compound was prepared according to methods described in steps E and F of Example 1. NMR($CD_3OD$): consistent with the desired structure; FAB mass spectral data: 836(M+H), 736(M-Boc+H)

EXAMPLE 9

Preparation of Boc-Phe-His-ACHPA-Lys-NH(2-oxoazepin-3-yl) hydrochloride [$N^{\alpha}$-t-butoxycarbonyl-L-phenylalanyl-L-histidyl-(3S,4S)-4-amino-5-cyclohexyl-3-hydroxypentanoyl-L-lysyl-L-3-amino-2-oxoazepine amide hydrochloride]

Step A: Boc-Lys(Cbz)-NH(2-oxoazepin-3-yl)

To a cooled (0°C) solution of Boc-Lys(Cbz)-OH (0.76 g, 2 mmols) in dry THF (10 ml), carbonyldiimidazole (0.324 g, 2 mmol) was added. After 1 hour of stirring at that temperature, the ice bath was removed and stirring continued for an additional 30 min. L-3-Aminocaprolactam (0.304 g, 2.4 mmol) was added and stirring continued at room temperature overnight. The mixture was then stirred with saturated solution of $NaHCO_3$ (25 ml) for 30 min., extracted with ethylacetate, and the organic phase was washed with water, 5% citric acid, water, dried ($MgSO_4$) and concentrated to dryness to give the product as a foam (0.85 g). TLC (ethylacetate): Rf 0.4; NMR($CDCl_3$): consistent with the desired structure; FAB mass spectral data: 981(2M+H), 491(M+H), 391(M-Boc+H).

Step B: Boc-Phe-His-ACHPA-Lys-NH(2-oxoazepin-3-yl) hydrochloride

Boc-Lys(Cbz)-NH(2-oxoazepin-3-yl) (0.25 g, 0.51 mmol) was treated with a sat. HCl in methanol (2 ml) at room temperature for 1 hour. Removal of the solvent and drying of the residue *in vacuo* gave Lys(CbZ)-NH(2-oxoazepin-3-yl) hydrochloride as a foam, which was coupled with Boc-Phe-His-ACHPA-$NHNH_2$ according to the procedure described in step E of Example 1, to give the fully protected peptide. The protected peptide was then subjected to catalytic hydrogenation condition described in step F of Example 1 to give the desired peptide as the hydrochloride. NMR($CD_3OD$): consistent with the desired structure; FAB mass spectral data: 838(M+H). $IC_{50}$ = 26.4nM.

EXAMPLE 10

Preparation of Boc-Phe-His-ACHPA-NHCH($CH_2NH_2$)CONHCH$_2$(pyridin-4-yl) hydrochloride [N$^{\alpha}$-t-butoxycarbonyl-L-phenylalanyl-L-histidyl-(3S,4S)-4-amino-5-cyclohexyl-3-hydroxypentanoyl-L-2,4-diaminopropionyl-4-(aminomethyl)pyridine amide hydrochloride]

Step A: Boc-NHCH($CH_2$NH-Cbz)CONHCH$_2$(pyridin-4-yl)

The title compound was prepared according to the procedure of Step A of Example 1, with Boc-NHCH-($CH_2$NHCbz)COOH replacing Boc-Lys(Cbz)-OH. The pure product was obtained as white solid (72%), after purification by MPLC on silica-gel using ethylacetate. TLC (EtOAC): Rf 0.26; NMR($CDCl_3$): consistent with the desired structure; FAB mass spectral data: 429(M+H), 329(M-Boc+H)

Step B: Boc-Phe-His-ACHPA-NHCH($CH_2NH_2$)CONHCH$_2$(pyridin-4-yl)hydrochloride

Boc-NHCH($CH_2$NH-Cbz)CONHCH$_2$(pyridin-4-yl) (0.753 g, 1.76 mmol) was deprotected with 50% TFA in $CH_2Cl_2$ (8 ml) to give $NH_2$CH($CH_2$NH-Cbz)CONHCH$_2$(pyridin-4-yl) bis-trifluoroacetate, which was coupled with the acylazide (obtained from Boc-Phe-His-ACHPA-NHNH$_2$) to give Boc-Phe-His-ACHPA-NHCH($CH_2$NH-Cbz)CONHCH$_2$(pyridin-4-yl). The product was purified by MPLC on silica-gel using ethylacetate-methanol-(9:1). NMR($CD_3OD$): consistent with the desired structure; FAB mass spectral data: 910(M+H),810(M-Boc+H).

The fully protected compound obtained above was subjected to catalytic hydrogenation condition described in step F of Example 1, to give the title compound. NMR($CD_3OD$): consistent with the desired structure; FAB mass spectral data: 776(M+H). $IC_{50}$ = 37nM

## EXAMPLE 11

Preparation of Boc-Phe-His-ACHPA-Lys-NHCH$_2$(piperidin-2-yl) dihydrochloride [N$^{\alpha}$-t-butoxycarbonyl-L-phenylalanyl-L-histidyl-(3S,4S)-4 amino-5-cyclohexyl-3-hydroxypentanoyl-L-lysyl-2-(aminomethyl)piperidine amide dihydrochloride]

The title compound is prepared from Boc-Phe-His-ACHPA-Lys(Cbz)-NHCH$_2$(pyridin-2-yl) according to the procedure described in Example 2.

## EXAMPLE 12

Preparation of Boc-Phe-His-ACHPA-Lys-NHCH$_2$CH$_2$N($CH_2CH_2$)$_2$O dihydrochloride [N$^{\alpha}$-t-butoxycarbonyl-L-phenylalanyl-L-histidyl-(3S,4S)-4-amino-5-cyclohexyl-3-hydroxypentanoyl-L-lysyl-N-(2-aminoethyl)-morpholine amide dihydrochloride]

The title compound was prepared according to the procedure of in Example 1, with Lys-NHCH$_2$CH$_2$N-($CH_2CH_2$)$_2$O replacing Lys-NHCH$_2$(pyridin-4-yl) as the C-terminal unit. FAB($CD_3OD$): consistent with the desired structure; FAB mass spectral data: 834(M+H), 734(M-Boc+H). $IC_{50}$ = 68.9nM

## EXAMPLE 13

Preparation of Boc-Phe-His-ACHPA-Lys-N($CH_2CH_2$)$_2$O hydrochloride [N$^{\alpha}$-t-butoxycarbonyl-L-phenylalanyl-L-histidyl-(3S,4S)-4-amino-5-cyclohexyl-3-hydroxypentanoyl-L-lysyl-N-morpholine amide hydrochloride]

Boc-Lys(Cbz)-$N(CH_2CH_2)_2O$ was prepared in 72% yield according to the procedure described in Step A of Example 1, with morpholine replacing 4-(aminomethyl)pyridine. The compound was then suitably deprotected and coupled according to the method in Step E of Example 1, to give Boc-Phe-His-ACHPA-Lys(Cbz)-$N(CH_2CH_2)_2O$, which was subjected to hydrogenolysis to give the title compound. NMR ($CD_3OD$): good for the desired structure; FAB mass spectral data: 854(M+H), 754(M-Boc+H). $IC_{50}$ = 289nM

## EXAMPLE 14

Preparation of Boc-Phe-His-ACHPA-Lys-$N(CH_3)CH_2$(piperidin-4-yl) dihydrochloride [$N^{\alpha}$-t-butoxycarbonyl-L-phenylalanyl-L-histidyl-(3S,4S)-4-amino-5-cyclohexyl-3-hydroxypentanoyl-L-lysyl-4-(methylaminomethyl)-piperidinamide dihydrochloride]

The title compound is prepared from Boc-Phe-His-ACHPA-Lys-$N(CH_3)CH_2$(pyridin-4 yl) according to the procedure described in Example 2.

## EXAMPLE 15

Preparation of Boc-Phe-His-ACHPA-Orn-$NHCH_2$(piperidin-4-yl) dihydrochloride [$N^{\alpha}$-t-butoxycarbonyl-L-phenylalanyl-L-histidyl- (3S,4S)-4-amino-5-cyclohexyl-3-hydroxypentanoyl-L-ornithinyl-4-(aminomethyl)-piperidinamide dihydrochloride]

Boc-Phe-His-ACHPA-Orn(Cbz)-$NHCH_2$(pyridin-4-yl) is prepared according to the procedure described in Example 1 using a suitable derivative of L-ornithine. The Cbz protecting is removed acoording to the method in Step F of Example 1 to give Boc-Phe-His-ACHPA-Orn-$NHCH_2$(pyridin-4-yl) as the hydrochloride. Boc-Phe-His-ACHPA- Orn(Cbz)-$NHCH_2$(pyridin-4-yl) is converted to the title compound according to the procedure in Example 2.

## EXAMPLE 16

Preparation of Boc-Phe-His-ACHPA-($N^{\epsilon}$,$N^{\epsilon}$-dimethyl)Lys-$NHCH_2$(pyridin-4-yl) [$N^{\alpha}$-t-butoxycarbonyl-L-phenylalanyl-L-histidyl-(3S,4S)-4-amino-5-cyclohexyl-3-hydroxypentanoyl-L-($N^{\epsilon}$,$N^{\epsilon}$-dimethyl)lysyl-4-(aminomethyl)pyridine amide]

Boc-Phe-His-ACHPA-Lys-$NHCH_2$(pyridin-4-yl) is dissolved in dry methanol and treated with 5 equivalents of HCHO (37% formalin), and the mixture is stirred with powdered 4Å molecular sieve at room temperature for 30 minutes. A solution of $NaCNBH_3$(3 equiv.) in methanol is added and stirring continued at room temperature for 18 hours. The mixture is filitered, and the filterate is evaporated to give the crude product. The pure product is obtained after purification of the above material by MPLC on LH-20.

## EXAMPLE 17

Preparation of Boc-Phe-His-ACHPA-Lys-$NHCH_2$(1-methylpiperidin-4-yl) [$N^{\alpha}$-t-butoxycarbonyl-L-phenylalanyl-L-histidyl-(3S,4S)-4-amino-5-cyclohexyl-3-hydroxy-pentanoyl-L-lysyl-4-(aminomethyl)-1-methylpiperidin amide]

A solution of Boc-Phe-His-ACHPA-Lys(Cbz)-$NHCH_2$(pyridin-4-yl)(0.24 g, 0.252 mmol) and $Et_3N$(0.035

ml) in DMF (2 ml) was treated with di-t-butyldicarbonate (0.056 g) at 0°C for 4 hours. The reaction mixture was poured into cold water and extracted with ethylacetate (3X 20 ml). The organic phase was washed with water, dried ($MgSO_4$) and conentrated to dryness in vacuo giving Boc-Phe-His(Boc)-ACHPA-Lys(Cbz)-$NHCH_2$(pyridin-4-yl) as glass like solid, which was used in the next step without further purification. NMR-($CD_3OD$) was found to be consistent with the desired structure.

A solution of Boc-Phe-His(Boc)-ACHPA-Lys(Cbz)-$NHCH_2$(pyridin-4-yl)(0.24 g, 0.228 mmol) in methanol (3 ml) was treated with $CH_3I$ (0.033 ml, 0.53 mmol) at room temperature overnight. The reaction mixture was concentrated in vacuo and the residue was treated with saturated $NH_3$ in methanol at room temperature for 5 hours. After removal of the solvent, the foam was purified on LH-20 column using methanol. The pure Boc-Phe-His-ACHPA-Lys(Cbz)-$NHCH_2$(1-methylpyridinium-4-yl)$^{\oplus}I^{\ominus}$ was obtained as a light yellow colored solid (0.15 g). The material gave an NMR spectrum consistent with the assigned structure. FAB mass spectral data: 966(M+)

Boc-Phe-His-ACHPA-Lys(Cbz)-$NHCH_2$(1-methyl-4-pyridinium)$^{\oplus}I^{\ominus}$ (0.12 g) was then hydrogenated according to the procedure in Example 2 to give the title compound.

## EXAMPLE 18

Preparation of Boc-Phe-His-ACHPA-Lys-$NHCH_2$(thiophen-2-yl) hydrochloride [$N^{\alpha}$-t-butoxycarbonyl-L-phenylalanyl-L-histidyl-(3S,4S)-4-amino-5-cyclohexyl-3-hydroxypentanoyl-L-lysyl-2-(aminomethyl)thiophene amide hydrochloride]

The title compound was prepared according to the procedure described in Example 1, with 2-(aminomethyl)thiophene replacing 4-(aminomethyl)pyridine. NMR($CD_3OD$): consistent with the desired structure. FAB mass spectral data: 823(M+H).

## EXAMPLE 19

Preparation of Boc-Phe-His-ACHPA-Lys-$NHCH_2CH_2$(1-methyl-pyrrol-2-yl) acetate [$N^{\alpha}$-t-butoxycarbonyl-L-phenylalanyl-L-histidyl-(3S,4S)-4-amino-5-cyclohexyl-3-hydroxypentanoyl-L-lysyl-2-(2-aminoethyl)-1-methylpyrrole amide acetate]

Boc-Lys(Cbz)-$NHCH_2CH_2$(1-methyl-2-pyrrole) was prepared according to the procedure of step A of Example 9 using 2-(2-amino)ethyl-1-methylpyrrole. The pure product was obtained in 76% yield, after purification of the crude product by MPLC on silica-gel using ethylacetate-hexane (2:1). NMR($CD_3OD$): consistent with the assigned structure. FAB mass spectral data: 487(M+H), 387(M-Boc+H).

The aforementioned compound was then converted to the title compound according to steps E and F of Example 1.

## EXAMPLE 20

Preparation of Boc-Phe-His-ACHPA-$N^{\alpha}(CH_3)$Lys-$NHCH_2$(pyridin-4-yl) acetate [$N^{\alpha}$-t-butoxycarbonyl-L-phenylalanyl-L-histidyl-(3S,4S)-4-amino-5-cyclohexyl-3-hydroxypentanoyl-$N^{\alpha}$-methyl-L-lysyl-4-(aminomethyl)pyridinamide acetate]

Boc-$N^{\alpha}$-$CH_3$-Lys(Cbz)-OH (obtained using intermediates prepared according to the procedure described by Freidinger et al., J. Org. Chem. 48, 77 (1983) is coupled with 4-(aminomethyl)pyridine as described in Step A of Example 1.

The title compound is prepared according to the procedure described in Example 1, with Boc-$N^{\alpha}$-$CH_3$-Lys-(Cbz)-$NHCH_2$(pyridin-4-yl) replacing Boc-Lys(Cbz)-$NHCH_2$(pyridin-4-yl).

## EXAMPLE 21

### Preparation of N-(morpholin-4-yl)carbonyl-Phenylalanine

To a suspension of phenylalanine methyl ester hydrochloride (1 gm, 0.00465 mol) in methylene chloride (10 ml) was added triethylamine (2 ml) followed by carbonyldiimidazole (0.83 g, 1 equiv.). After stirring for 1 hour at room temperature, morpholine (0.8 ml, 2 equiv.) was added and the reaction was stirred a further 2 hours until TLC (silica, EtOAc) showed the reaction to be complete. The reaction mixture was washed 1 time with HCl (IN), dried over $Na_2SO_4$, then filtered and evaporated in vacuo. The product was purified by chromatography (silica, EtOAc) to give N-(morpholin-4-yl)carbonylphenylalanine methyl ester (1 g). TLC-(silica,EtOAc) Rf= 0.52. NMR($CDCl_3$) 3.1(m,2H), 3.25-3.35(m,4H), 3.65-3.7(m,4H), 3.75(s,3H), 4.75-4.85-(m,1H), 7.1(d,2H), 7.3(m,3H).

To a solution of the aforementioned material (0.485 g) in methanol (3 ml) at room temperature was added a solution of KOH (1N, 5.3 ml). After stirring for 3 hours the reaction mixture was acidified with HCL (3N), and extracted with methylene chloride. The combined organic fractions were dried as usual, filtered and evaporated to driness to give 0.39 g of the title compound. NMR($CDCl_3$) 3.2(m,2H), 3.25-3.35(m,4H), 3.35-3.45(m,4H), 4.75-4.86(m,1H), 7.2(d,2H), 7.25-7.35(m,3H).

## EXAMPLE 22

### N-(4-Boc-piperazinyl)carbonyl-Phenylalanine

This compound was prepared according to the procedures outlined in Example 21 using N-Boc-piperazine, phenylalanine methyl ester, and carbonyldiimidazole. NMR($CDCl_3$) 1.45(s,9H), 3.1-3.2(2d,2H), 3.3-3.4(m,4H), 3.3-3.45(m,4H), 4.55-4.65(m,1H), 4.8(d,1H), 7.2(d,2H), 7.25-7.35(m,3H).

## EXAMPLE 23

### Preparation of N-[(Morpholin-4-yl)carbonyl]Phe-His-ACHPA-Lys-$NHCH_2$(pyridin-4-yl)

#### Step A: N-[(Morpholin-4-yl)carbonyl)Phe-His-ACHPA-Lys-$NHCH_2$(pyridin-4-yl)

Boc-His(DNP)-ACHPA-Lys(Cbz)-$NHCH_2$(pyridin-4-yl) was deprotected according to the procedure described in step C of Example 6, and was coupled with N-(morpholin-4-yl)carbonyl-phenylalanine in presence of BOP reagent (benzotriazol-1-yloxy tris(dimethylamino)phophonium hexafluorophosphate) in methylene chloride at room temperature (18 hours). The coupled product was isolated, and was redissolved in methylenechloride and treated with thiophenol as described in step D of Example 6.

The crude product was purified on LH-20 using methanol, to give the title compound (0.22 g) as a foam. TLC(silica,80:15:1 $CHCl_3$:MeOH:$NH_3$) Rf=0.38. FAB mass spectral data: 965(M+H). NMR is consistent with structure.

#### Step B: N[(Morpholin-4-yl)carbonyl]Phe-His-ACHPA-Lys-$NHCH_2$(pyridin-4-yl)

The compound from Step A, in methanol was added to a solution of HBr(30%) in AcOH at 0°C. The mixture was stirred at that temperature for 15 minutes and at room temperature for an additional 20 minutes. The reaction was evaporated to dryness in vacuo. The residue was dissolved in methanol and treated with propylene oxide to give a white precipitate, which was filtered, washed and dried in vacuo. The title compound (0.15 g) was purified by HPLC (RP-18,1:1 MeOH:$H_2O$) rt=2.52 min. FAB mass spectral

data: 831(M + H). NMR was consistent with the assigned structure. $IC_{50}$ = 12nM.

## EXAMPLE 24

### Preparation of N-[(2-t-butoxycarbonylamino)ethoxycarbonyl]-L-phenylalanine

3.60 g (16.7 mmol) of L-phenylalanine methyl ester hydrochloride (Sigma) was suspended in 5 mL dry DMF at 0° C and treated with 3.36 g (27.5 mmol, 1.6 eq) 4-dimethylaminopyridine followed by 4.43 g (27.3 mmol, 1.6 eq) carbonyldiimidazole. After 30 minutes at 0° C and 1 hour at room temperature, a solution of 4.162 g (25.8 mmol, 1.5 eq) N-BOC-2-aminoethanol (Sigma) in 3 mL dry DMF was added and the mixture was heated at 90° C for 2 hours, then cooled and diluted with 100 mL ethyl acetate.

The solution was washed with 0.1N HCl (4x), 5% $NaHCO_3$ (1x) and brine (1x); dried over $MgSO_4$, and filtered with volatiles being removed in vacuo. The crude product was purified by medium pressure liquid chromatography, eluting with hexanes/ethyl acetate (1:1) to afford 4.42 g (12.1 mmol, 72%) of the methyl ester of the title compound as a viscous oil.

The product obtained above was dissolved in 12 mL methanol and treated with 13.3 ml of 1.0M NaOH (13.3 mmol, 1.1 eq) at room temperature. After 2.5 hours, an additional 3.0 mL of 1.0M NaOH (0.25 eq) was added and the mixture was maintained at room temperature for another hour.

The reaction mixture was filtered, concentrated in vacuo, diluted with 20 mL distilled water, cooled to 0°, and acidified (pH 2, paper) by the dropwise addition of saturated aqueous $KHSO_4$. The resulting mixture was then extracted with ethyl acetate (4x), and the combined extracts washed with 5% $NaHCO_3$ (1x) and brine (1x), then dried over $MgSO_4$, and filtered, with the solvent being removed in vacuo to afford 3.98 g (11.3 mmol, 93%) of the title compound as a white solid.

$R_f$ ($CHCl_3/CH_3OH/H_2O/HOAc$; 100:20:3:.5) 0.42. NMR (300MHz, $CD_3OD$): 7.2(m,5H), 4.40 (dd,1H), 3.95 (m,2H), 3.2 (m,3H), 2.93(dd,1H), 1.42(s,9H). FAB-MS: 353 (M + H,10%), 253 (M-BOC + H,100%).

## EXAMPLE 25

### Preparation of N-(2-propylsulfonyl)-L-phenylalanine

8.0 g (37 mmol) of L-phenylalanine methyl ester (Sigma) was suspended in 35 mL $CH_2Cl_2$ and the mixture was cooled to 0° under a nitrogen atmosphere. Isopropylsulfonyl chloride (Aldrich) (4.57 mL, 5.80 g, 40.7 mmol, 1.1 eq) was added, followed by dropwise addition of 18 mL triethylamine (13.1 g, 130 mmol, 3.5 eq) over 15 minutes. The resulting mixture was gradually warmed to room temperature and stirred at that temperature for 18 hours, at which time an additional 2.0 mL (0.5 eq) of isopropylsulfonyl chloride was added.

After 3 more hours at room temperature, the mixture was filtered, diluted with EtOAc, washed with 1N HCl (3x), 5% $NaHCO_3$ (3x) and brine (1x), then dried over $MgSO_4$, filtered and stripped. The residue was purified by medium pressure liquid chromatography, eluting with hexanes/ethylacetate (3:1) to afford 2.56 g (8.98 mmol, 24%) of the methyl ester of the title compound as a viscous oil.

0.669 g (2.34 mmol) of the above compound was dissolved in 5 mL of methanol and treated with 2.8 mL of 1.0M NaOH (2.8 mmol, 1.2 eq). After one hour at room temperature, the mixture was concentrated in vacuo, diluted with water, cooled to 0° C and acidified by dropwise addition of 6N HCl. The resulting mixture was then extracted with ethyl acetate (3x) and the combined extracts washed with brine (1x), dried over $MgSO_4$, filtered and stripped to afford the title compound (0.568 g, 2.10 mmol, 89%) as a tan solid. $R_f$ ($CHCl_3/MeOH/H_2O/HOAc$; 100:20:3:.5): 0.49. FAB-MS: 272 (M + H,100%). NMR (300MHz,$CD_3OD$): 7.3-(m,5H), 4.13(dd,1H), 3.19(dd,1H), 2.87(dd,1H), 2.80(m,1H), 1.15(d,3H), 1.02(d,3H).

## EXAMPLE 26

Preparation of Boc-$NHCH_2CH_2OCO$-Phe-His-ACHPA-Lys-$NHCH_2$(piperidin-4-yl)

200 mg (0.21 mmol) of Boc(DNP)His-ACHPA-(Cbz)Lys-$NHCH_2$(pyridin-4-yl) (as prepared in Example 6) was dissolved in 1.5 mL of methanol and treated with 1.5 mL of HCl saturated methanol solution. After 1.5 hours, TLC ($CHCl_3$/MeOH/$H_2O$/HOAc 100:20:3:.5) showed complete disappearance of starting material. All volatiles were removed in vacuo and the residue was further evacuated under high vacuum.

The residue was redissolved in 3 mL of dry $CH_2Cl_2$, cooled to 0° C, and treated with 80 mg (0.23 mmol, 1.1 eg) of the acid prepared as described in Example 24, 90 µL diisopropylethylamine (67 mg, 0.52 mmol, 2.5 eq) and 185 mg (0.42 mmol, 2 eq) BOP reagent. After 5 hours at 0° C an additional 50µL (1.2 eq) diisopropylethylamine and 46 mg (0.5 eq) BOP were added, and the reaction was maintained at 0° C overnight.

The reaction mixture was diluted with ethyl acetate and washed with pH 7.0 phosphate buffer (1x), 5% $NaHCO_3$ (2x) and brine (1x), then dried over $MgSO_4$ and filtered, with volatiles being removed in vacuo. The crude product was purified by reverse- phase HPLC ($C_{18}$), eluting with MeOH/$H_2O$ (85:15), to afford 96 mg (0.080 mmol, 38%) of the product as a pale yellow powder. FAB-MS: 1205 (M+H,24%).

The compound thus prepared (96 mg, 0.080 mmol) was dissolved in 1 mL of $CH_2Cl_2$ and treated with 41µL (44 mg, 0.4 mmol, 5 eq) of thiophenol. The mixture was stirred 18 hours at room temperature, at which time all volatiles were removed in vacuo, and the residue was dissolved in ethyl acetate and washed with 5% $NaHCO_3$ (5x) and brine (2x), then dried over $MgSO_4$, filtered and stripped of solvent under vacuum. The residue was purified by RP-HPLC ($C_{18}$), eluting with MeOH/$H_2O$ (80:20) to afford 25 mg (0.024 mmol, 30%) of the product as a white powder. FAB-MS; 1039 (M+H, 100%).

This compound (14 mg, 0.013 mmol) was dissolved in 3 mL of MeOH with 2.1 equivalents of HCl added, and hydrogenated at 40psi over 14 mg 10% Pd/C overnight. The mixture was filtered through a pad of Celite and volatiles were removed under vacuum.

The title compound (10 mg, 0.010 mmol, 78%) was thus obtained as a tan powder.

$R_f$ (EtOAc/BuOH/HOAc/$H_2O$; 1:1:1:1); 0.4 (elongated). FAB-MS; 911 (M+H, 100%). Partial NMR (300MHz, $CD_3OD$) 8.83(s,1H), 7.38(s,1H), 7.2(m,5H), 4.67(m,1H), 4.26(m,1H), 4.0(m,2H), 2.37(d,2H), 1.40(s,9H). $IC_{50}$ = 39nM.

## EXAMPLE 27

i-Pr$SO_2$-Phe-His-ACHPA-Lys-$CH_2$(piperidin-4-yl)

200 mg (0.206 mmol) BOC-His(DNP)-ACHPA-(Cbz)Lys-$NHCH_2$(pyridin-4-yl), as prepared in Example 6, was dissolved in 1.5 mL of MeOH and treated with 1.5 mL of HCl-saturated methanol solution at room temperature. After 2.5 hours, all the volatiles were removed in vacuo and the residue was further evacuated under high vacuum.

The residue was redissolved in 3 mL of $CH_2Cl_2$, cooled to 0° C and treated with 60 mg (0.221 mmol, 1.1 eq) of the sulfonic acid prepared in Example 25, 90µL (67 mg, 0.52 mmol, 2.5 eq) of diisopropylethylamine and 185 mg (0.42 mmol, 2 eq) of BOP reagent. After 2 hours at 0° C, the reaction mixture was warmed to room temperature and an additional 50µL (1.4 eq) of diisopropylethylamine and 46 mg (0.5 eq) of BOP reagent were added, and the reaction was maintained at room temperature for 3 hours.

The mixture was diluted with ethyl acetate and washed with 5% $NaHCO_3$ (5x), pH 7.0 phosphate buffer (1x) and brine (1x), then dried over $MgSO_4$ and filtered, with volatiles being removed in vacuo. The crude product was purified by reverse-phase HPLC ($C_{18}$), eluting with MeOH/$H_2O$ (85:15), to afford 117 mg (0.11 mmol, 53%) of the product as a yellow powder.

This product (117 mg, 0.11 mmol) was dissolved in 2 mL of $CH_2Cl_2$ and treated with 56µL (60 mg, 0.55 mmol, 5 eq) of thiophenol at room temperature. After stirring at room temperature for 18 hours, the reaction mixture was diluted into ethyl acetate and washed with 5% $NaHCO_3$ (5x) and brine (1x), then dried over $MgSO_4$ and filtered with volatiles being removed in vacuo. The residue was purified by reverse-phase HPLC, eluting with MeOH/$H_2O$, to afford 75 mg (0.078 mmol, 72%) of the product as a tan powder. FAB-MS; 958 (M+H,100%).

37 mg (0.038 mmol) of the above intermediate in 3 mL of MeOH was hydrogenated over 37 mg of 20% palladium hydroxide at 40psi, with 2.1 eq HCl added. After five hours, the hydrogenation was stopped and the reaction mixture was filtered through a pad of Celite, with the filtrate being stripped of volatiles under

vacuum.

The title compound (30 mg, 0.033 mmol, 86%) was obtained as a tan powder, homogeneous by TLC. Rf (EtOAc/BuOH/HOAc/ $H_2O$; 1:1:1:1) 0.45. FAB-MS; 830 (M+H,100%). Partial NMR (300MHz,$CD_3OD$); 8.80(s,1H), 7.40(s,1H), 7.25(m,5H), 7.73(dd,1H), 4.25(dd,1H), 4.0(m,2H), 2.38(d,3H), 1.04(d,3H), 0.78(d,3H). $IC_{50}$ = 159nM

## Claims

1. A peptide of the formula:

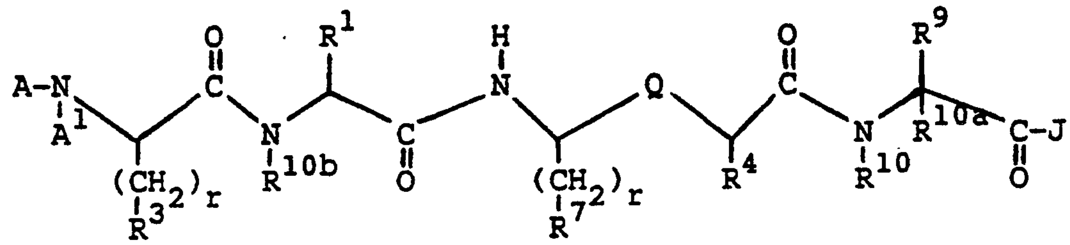

(I),

wherein:

A is hydrogen;

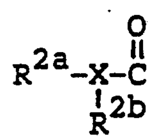

,

where

X is -O-; -O-CH-; -CH-O-; -CH-; N-;-NH-CH-; or-S-CH-; and

$R^{2a}$ and $R^{2b}$ are independently hydrogen or unsubstituted or mono- or disubstituted $C_1$-$C_4$-alkyl, wherein the substituent(s) is/are independently selected from the group consisting of amino; mono- or di-$C_1$-$C_4$-alkylamino; t-butoxycarbonylamino; acetylamino; carbobenzyloxyamino; trimethylacetylamino; t-butylacetylamino; hydroxy; $-SO_3H$; $-CO_2H$;$-CO_2$-$C_1$-$C_4$-alkyl; $C_1$-$C_4$-alkyl-$SO_2$; aryl, where aryl is unsubstituted or mono-, di- or trisubstituted phenyl or naphthyl, wherein the substituent(s) is/are independently selected from the group consisting of $C_1$-$C_8$-alkyl, amino, mono- or di-$C_1$-$C_4$-alkylamino, amino-$C_1$-$C_4$-alkyl, hydroxy-$C_1$-$C_4$-alkyl, phenyl-$C_1$-$C_4$-alkyl, mono- or di-$C_1$-$C_4$-alkylamino-$C_1$-$C_4$-alkyl, guanidyl, guanidyl-$C_1$-$C_4$-alkyl, hydroxyl, $C_1$-$C_4$-alkoxy, $CF_3$, halo, CHO, $CO_2H$, $CO_2$-$C_1$-$C_4$-alkyl, $CO_2$-$C_1$-$C_4$-alkoxy, $NR^5R^6$, and $N(R^5)_3^{\oplus} A^{\ominus}$, wherein $R^5$ and $R^6$ are independently hydrogen, unsubstituted or monosubstituted $C_1$-$C_4$-alkyl, where the substituent is amino, mono- or di-$C_1$-$C_4$-alkylamino, hydroxyl, $C_1$-$C_4$-alkoxy or $N(C_1\text{-}C_4\text{-alkyl})_3^{\oplus} A^{\ominus}$; and $A^{\ominus}$ is a counterion selected from the group consisting of single negatively-charged ions; $-CONR^5R^6$, where $R^5$ and $R^6$ are as defined above; and Het, where Het is an unsubstituted or mono- or disubstituted 5-to 7-membered mono- or bicyclic or 7- to 10-membered bicyclic heterocyclic ring, wherein the one or two heteroatoms are independently selected from the group consisting of N, O, S, NO, SO, $SO_2$ and quaternized N, and the substituent(s) is/are independently selected from the group consisting of hydroxy, thiol, $C_1$-$C_6$-alkyl, $CF_3$, $C_1$-$C_4$-alkoxy, halo, aryl, as defined above, aryl-$C_1$-$C_4$-alkyl, amino, mono- or di-$C_1$-$C_4$-alkylamino, amino-$C_1$-$C_4$-alkyl, hydroxy-$C_1$-$C_4$-alkyl, di-$C_1$-$C_4$-alkylamino-$C_1$-$C_4$-alkyl, guanidyl, guanidyl- $C_1$-$C_4$-alkyl, $CO_2H$, $CO_2$-$C_2$-$C_4$-alkyl, $NR^5R^6$ and $N(R^5)_3^{\oplus} A^{\ominus}$, wherein $R^5$, $R^6$, and $A^{\ominus}$ are as defined above, or when the heteroatom N is present, the substituents are alternatively-$(CH_2)_q$- or $-(CH_2)_2$-O-$CH_2)_2$- and form a quaternary spirocyclic ring with the N-atom, wherein q is 3-to-6, except that where X is -O-, only one of $R^{2a}$ or $R^{2b}$ is present;

$R^{2c}SO_2$-,

where

$R^{2c}$ is unsubstituted, mono- or di-substituted $C_1$-$C_4$-alkyl wherein the substituent(s) is/are independently

selected from the group consisting of amino, mono- or di-$C_1$-$C_4$-alkylamino, t-butoxycarbonylamino, carbobenzyloxyamino, acetylamino, trimethylacetylamino, hydroxy, -$SO_3H$, -$CO_2H$, -$CO_2$-$C_1$-$C_4$-alkyl, $C_1$-$C_4$-alkyl-$SO_2$- and Het, as defined above; or

Het-CO, where Het is as defined above;

$A^1$is hydrogen or $C_1$-$C_4$-alkyl;

r is 1-to-4;

$R^3$ is hydrogen, $C_1$-$C_4$-alkyl, aryl, as defined above, aryl $C_1$-$C_4$-alkyl, or indolyl;

$R^1$ is hydrogen;

aryl, as defined above;

Het-S-$CH_2$;

Het-SO-$CH_2$;

Het-SO-$CH_2$; or

unsubstituted or monosubstituted $C_1$-$C_4$-alkyl or $C_1$-$C_4$-alkylthio-$C_1$-$C_4$-alkyl, where the substituent is on the terminal carbon atom and is selected from the group consisting of $C_1$-$C_4$-alkoxy, amino, mono- or di-$C_1$-$C_4$-alkylamino, carboxy, $CO_2$-$C_1$-$C_4$-alkyl, aryl, as defined above, Het, as defined above, or -$CONR^5R^6$, wherein $R^5$ and $R^6$ are as defined above;

$R^7$ is $C_3$-$C_6$-alkyl;

aryl, as defined above; or

unsubstituted or mono-, di- or trisubstituted $C_3$-$C_7$-cycloalkyl, wherein the substituent(s) is/are independently selected from the group consisting of $C_1$-$C_4$-alkyl, trifluoromethyl, hydroxy, $C_1$-$C_4$-alkoxy and halo;

Q is

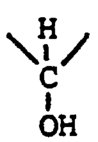 ; 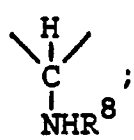 ; 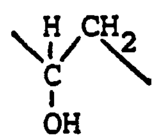 ; or 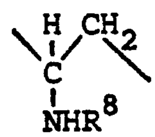 ,

where

$R^8$ is $C_1$-$C_4$-alkyl; formyl; $C_1$-$C_4$-alkanoyl; aroyl; $C_1$-$C_4$-alkoxycarbonyl; aryloxycarbonyl; or aryl-$C_1$-$C_4$-alkoxycarbonyl, wherein aryl is as defined above;

$R^4$ is hydrogen;

$C_1$-$C_8$-alkyl;

$C_2$-$C_8$-alkenyl;

mono- or disubstituted $C_2$-$C_8$-alkyl, wherein the substituent(s) is/are on the final 1 or/and 2 carbon atoms of the alkyl chain and is/are independently selected from the group consisting of hydroxy, carboxy, $CO_2$-$C_1$-$C_4$-alkyl, amino, mono-, di- or trialkylamino, guanidyl, $NR^5R^6$ and $N(R^5)_3^{\oplus} A^{\ominus}$, where $R^5$, $R^6$ and $A^{\ominus}$ are as defined above;

aryl, as defined above; or

unsubstituted, mono-, di- or trisubstituted $C_3$-$C_7$-cycloalkyl, wherein the substituent(s) is/are independently selected from the group consisting of $C_1$-$C_4$-alkyl, trifluoromethyl, hydroxy, $C_1$-$C_4$-alkoxy and halo;

$R^9$ is -$(CH_2)_s$-$NR^{11}R^{12}$, where $R^{11}$ and $R^{12}$ are independently hydrogen; Het, as defined above; aryl, as defined above; $C_3$-$C_7$-cycloalkyl; or unsubstituted or monosubstituted $C_1$-$C_4$-alkyl, where the substituent is on the terminal carbon atom and is chosen from the group consisting of aryl, as defined above; Het, as defined above; amino; hydroxyl; mono- or di-$C_1$-$C_4$-alkylamino; $CO_2H$; $CONR^5R^6$, wherein $R^5$ and $R^6$ are as defined above; and $SO_2H$; and s is 1-to-5;

-$(CH_2)_tN(R^{11})_3^{\oplus} A^{\ominus}$, where t is 2-to-5; and $R^{11}$ and $A^{\ominus}$ are as defined above;

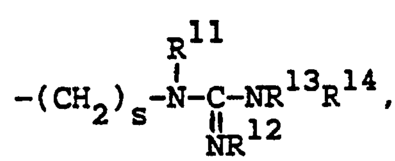 ,

where $R^{13}$ and $R^{14}$ are independently chosen from the definitions of $R^{11}$, or together are -$(CH_2)_4$- or -$(CH_2)_5$-; and $R^{11}$, $R^{12}$ and s are as defined above;

-$(CH_2)_5N=CH$-$NR^{13}R^{14}$, where s, $R^{13}$ and $R^{14}$, or $R^{13}$ and $R^{14}$ taken together, are as defined above; or

$(CH_2)_s$-$R^{18}$,

where $R^{18}$ is -$CH(CH_2CH_2)_2NR^{10}$,

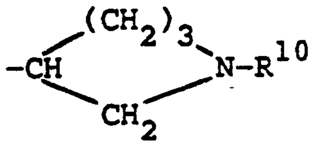

or

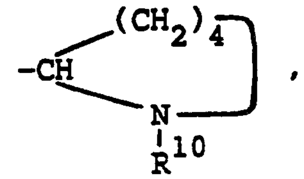

wherein $R^{10}$ is H or $C_1$-$C_4$-alkyl; and s is as defined above;
$R^{10a}$ and $R^{10b}$ are independently H or $C_1$-$C_4$-alkyl;

J is NH- $\overset{R^{15}}{CH}$ $-(CH_2)_u-R^{16}$, where $R^{15}$ is hydrogen, $C_1$-$C_4$-alkyl or aryl-$C_1$-$C_4$-alkyl, wherein aryl is as defined above; u is 0-to-4; and $R^{16}$ is Het, as defined above; monosubstituted phenyl or naphthyl, wherein the substituent is selected from the group consisting of $C_1$-$C_8$-alkyl, amino, mono- or di-$C_1$-$C_4$-alkylamino, amino-$C_1$-$C_4$-alkyl, hydroxy-$C_1$-$C_4$-alkyl, mono- or di-$C_1$-$C_4$-alkylamino-$C_1$-$C_4$-alkyl, guanidyl, guanidyl-$C_1$-$C_4$-alkyl, hydroxyl, $C_1$-$C_4$-alkyl, $CF_3$, halo, CHO, $CO_2H$, $CO_2$-$C_1$-$C_4$-alkoxy, $CO_2$-$C_1$-$C_4$-alkoxy, $NR^5R^6$ and $N(R^5)_3^{\oplus}A^{\ominus}$, where $R^5$, $R^6$ and $A^{\ominus}$ are as defined above; or when u is not equal to 0, also alternatively, $-NR^{11}R^{12}$ or $-N(R^{11}R^{12}R^{13})^{\oplus}A^{\ominus}$, wherein $R^{11}$, $R^{12}$, $R^{13}$ and $A^{\ominus}$ are as defined above;

-O- $\overset{R^{15}}{CH}$ $-(CH_2)_u-R^{16}$, where $R^{15}$, $R^{16}$ and u are as defined above;

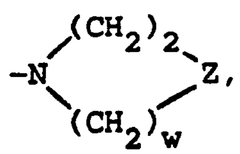

where Z is $-NR^{11}$, wherein $R^{11}$ is as defined above;

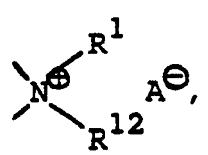

wherein $R^1$, $R^{12}$ and $A^{\ominus}$, and are as defined above; O; S; SO; or $SO_2$; and w is 2 or 3;
-NH-Het, where Het is as defined above;

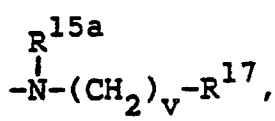

where v is 0-to-4; $R^{17}$ is Het, as defined above; aryl, as defined above; $C_1$-$C_4$-alkyl; or, when v is not equal to 0 or 1, alternatively $NH_2$, $NR^{11}R^{12}$, $N(R^{11}R^{12}R^{13})^{\oplus}A^{\ominus}$, hydroxyl, or $SO_3H$, wherein $R^{11}$, $R^{12}$, $R^{13}$, and $A^{\ominus}$ are as defined above; and $R^{15a}$ is $C_1$-$C_4$-alkyl or aryl $C_1$-$C_4$-alkyl, wherein aryl is as defined above; or, when v is not equal to 0, also alternatively $CO_2H$, $CO_2$-$C_1$-$C_4$-alkyl or $CONR^5R^6$, wherein $R^5$ and $R^6$ are as defined above;

$$\begin{array}{c} NR^{12} \\ | \\ -NR^{11}-C-NR^{13}R^{14}, \end{array}$$

where $R^{11}$, $R^{12}$, $R^{13}$ and $R^{14}$ are as defined above; or
$-N=CH-NR^{11}R^{12}$, where $R^{11}$ and $R^{12}$ are as defined above;
or a pharmaceutically-acceptable salt thereof.

2. A peptide according to Claim 1, selected from the list consisting of:
Boc-Phe-His-ACHPA-Lys-$NHCH_2$(pyridin-4 yl)
Boc-Phe-His-ACHPA-Lys-$NHCH_2$(pyridin-2-yl)
Boc-Phe-His-ACHPA-($N^{\alpha}$-Me)Lys-$NHCH_2$(pyridin-4-yl)
Boc-Phe-His-ACHPA-($\alpha$-Me)Lys-$NHCH_2$(pyridin-4-yl)
Boc-Phe-His-ACHPA-Lys-$N(CH_3)CH_2$(pyridin-4-yl)
Boc-Phe-His-ACHPA-($N^{\alpha}$-Me)Lys-$N(CH_3)CH_2$(pyridin-4-yl)
Boc-Phe-His-ACHPA-Lys-$NHCH_2$(piperidin-4-yl)
Boc-Phe-His-ACHPA-Lys-$NHCH_2$(piperidin-2-yl)
Boc-Phe-His-ACHPA-($N^{\alpha}$-Me)Lys-$NHCH_2$(piperidin-4-yl)
Boc-Phe His-ACHPA-($\alpha$-Me)Lys-$NHCH_2$(piperidin-4-yl)
Boc-Phe-His-ACHPA-Lys-$N(CH_3)CH_2$(piperidin-4-yl)
Boc-Phe-His-ACHPA-Lys-$NHCH_2$(1-methylpiperidin-4-yl)
Boc-Phe-His-ACHPA-Lys-$NHCH_2$(1-benzylpiperidin-4-yl)
Boc-Phe-His-ACHPA-Lys-$NHCH_2$(1-carboxymethylpiperidin-4-yl)
Boc-Phe-His-ACHPA-($N^{\alpha}$-Me)Lys-$N(CH_3)CH_2$(piperidin-4-yl)
[Boc-Phe-His-ACHPA-Lys-$NHCH_2$(1-methylpyrid-4-yl)]$^{\oplus}$ OAc$^{\ominus}$
Boc-Phe-His-ACHPA-($N^{\epsilon}$,$N^{\epsilon}$-dimethyl)Lys-$NHCH_2$ (-pyridin-4-yl)
Boc-Phe-His-ACHPA-($N^{\epsilon}$-isopropyl)Lys-$NHCH_2$(pyridin-4-yl)
Boc-Phe-His-ACHPA-Orn-$NHCH_2$(pyridin-4-yl)
Boc-Phe-His-ACHPA-$NH(CH_2NH_2)CO$-$NHCH_2$(pyridin-4-yl)
Boc-Phe-His-ACHPA-($N^{\epsilon}$,$N^{\epsilon}$-diethyl)-Lys-$N(CH_3)$ $CH_2$(pyridin-4-yl)
Boc-Phe-His-ACHPA-Lys-NH(3-quinuclidinyl)
[Boc-Phe-His-ACHPA-Lys-NH(N-methyl-3-quinuclidinyl)]$^{\oplus}$ Cl$^{\ominus}$
[Boc-Phe-His-ACHPA-($N^{\epsilon}$,$N^{\epsilon}$,$N^{\epsilon}$-trimethyl)-Lys-$NHCH_2$(pyridin-4-yl]$^{\oplus}$ Cl$^{\ominus}$
Boc-Phe-His-ACHPA-($N^{\epsilon}$-benzyl)Lys-$NHCH_2$(pyridin-4-yl)
Boc-(p-OMe)Phe-His-ACHPA-Lys-$NHCH_2$(pyridin-4-yl)
Boc-(p-OMe)Phe-His-ACHPA-Lys-NH(3-quinuclidinyl)
Boc-(p-OMe)Phe-His-ACHPA-($N^{\epsilon}$,$N^{\epsilon}$-diethyl)-Lys-$NHCH_2$(pyridin-4-yl)
Boc-Phe-His-ACHPA-$NHCH[(CH_2)_4NHC(=NCH_3)NHCH_3]CO$-$NHCH_2$(pyridin-4-yl)
Boc-Phe-His-ACHPA-($N^{\epsilon}$-imidazolin-2-yl)Lys-$NHCH_2$(pyridin-4-yl)
Boc-Phe-His-ACHPA-Lys-$NHCH_2$(2-butyl)$CH_2N(CH_3)_3^{\oplus}$ OAc$^{\ominus}$
Boc-Phe-His-Cal[$CH(OH)CH_2$]Val-Lys-$NHCH_2$(pyridin-4-yl)
Boc-Phe-His-Cal[$CH(OH)CH_2$]Val-($\alpha$-Me)Lys-$NHCH_2$(pyridin-4-yl)
Boc-Phe-His-Cal[$CH(OH)CH_2$]Val-Lys-$N(CH_3)CH_2$(pyridin-4-yl)
Boc-Phe-His-Cal[$CH(OH)CH_2$]Val-Lys-$N(CH_2CH_2)_2O$
[Boc-Phe-His-Cal[$CH(OH)CH_2$]Val-Lys-$N(CH_2CH_2(OH)$
[Boc-Phe-His-Cal[$CH(OH)CH_2$]Val-Lys-$NHCH_2CH_2N(CH_2CH_2)_2O(CH_3)$$^{\oplus}$Cl$^{\ominus}$
Boc-Phe-($N^{\alpha}$-Me)His-Cal[$CH(OH)CH_2$]Val-Lys-$NHCH_2$ (pyridin-4-yl)
Boc-Phe-($N^{\alpha}$-Me)His-Cal[$CH(OH)CH_2$]Val-($\alpha$-Me)Lys-$NHCH_2$(pyridin-4-yl)
Boc-Phe-($N^{\alpha}$-Me)His-Cal[$CH(OH)CH_2$]Val-Lys-$N(CH_3)$ $CH_2$(pyridin-4-yl)
Boc-Phe-($N^{\alpha}$-Me)His-Cal[$CH(OH)CH_2$]Val-Lys-$N(CH_2CH_2)_2O$
$HO_2C(CH_2)_4CO$-Phe-His-ACHPA-Lys-$NHCH_2$(pyridin-4-yl)
$HO_2C(CH_2)_4CO$-Phe-His-ACHPA-Lys-$NHCH_2$(piperidin-4-yl)
$HO_2CCH_2C(CH_3)_2CO$-Phe-His-ACHPA-Lys-$NHCH_2$(piperidin-4-yl)
$O(CH_2CH_2)_2NCO$-Phe-His-ACHPA-Lys-$NHCH_2$(pyridin-2-yl)
$iPrSO_2$-Phe-His-ACHPA-Lys-$NHCH_2$(pyridin-4-yl)
$BocNHCH_2CH_2OCO$-Phe-His-ACHPA-Lys-$NHCH_2$(pyridin-4-yl)
$(CH_3)_2N(CH_2)_3CO$-Phe-His-ACHPA-Lys-$NHCH_2$(pyridin-4-yl)

$O(CH_2CH_2)_2NCO$-($N^{\alpha}$-Me)Phe-His-ACHPA-Lys-$NHCH_2$(pyridin-4-yl)
$CH_3N(CH_2CH_2)_2NCO$-Phe-His-ACHPA-Lys-$NHCH_2$(pyridin-2-yl)
$SO_2(CH_2CH_2)_2NCO$-Phe-His-ACHPA-Lys-$NHCH_2$(pyridin-2-yl)
$SO_2(CH_2CH_2)_2NCO$-($N^{\alpha}$-Me)Phe-His-ACHPA-Lys-$NHCH_2$ (piperidin-4-yl)
$HO_2CCH_2N(CH_2Ch_2)_2NCO$-Phe-His-ACHPA-Lys-$NHCH_2$(pyridin-4-yl)
$HO_2CCH_2CH_2N(CH_2CH_2)_2NCO$-Phe-His-ACHPA-Lys-$NHCH_2$ (pyridin-4-yl)
$HO_2CCH_2(CH_3)NCO$-Phe-His-ACHPA-Lys-$N(CH_3)CH_2$(piperidin-4-yl)
$O(CH_2CH_2)_2NCH_2CH_2SO_2$-Phe-His-ACHPA-Lys-$N(CH_3)CH_2$ (pyridin-4-yl)
Boc-Phe-His-(2S-isobutyl)ACHPA-Lys-$NHCH_2$(pyridin-4-yl)
Boc-Phe-His-AmACHPA-Lys-$NHCH_2$(pyridin-2-yl)
Boc-Phe-His-(2S-allyl)ACHPA-($N^{\alpha}$-Me)Lys-$NHCH_2$(pyridin-4-yl)
Boc-Phe-His-(2S-ethyl)ACHPA-(α-Me)Lys-$NHCH_2$(pyridin-4-yl)
Boc-Phe-His-(2S-n-butyl)ACHPA-Lys-$N(CH_3)CH_2$(pyridin-4-yl)
Boc-Phe-His-AmACHPA-($N^{\alpha}$-Me)Lys-$N(CH_3)CH_2$(pyridin-4-yl)
Boc-Phe-His-AmACHPA-Lys-$NHCH_2$(pyridin-4-yl)
Boc-$NHCH_2CH_2O$-CO-Phe-His-ACHPA-Lys-$NHCH_2$(piperidin-4-yl)
$O(CH_2CH_2)_2NCO$-Phe-His-ACHPA-Lys-$NHCH_2$(piperidin-4-yl);
and
i-$PrSO_2$-Phe-His-ACHPA-Lys-$NHCH_2$(piperidin-4-yl).

3. A peptide of the formula, A-B-D-G-T-J, wherein A is Boc, EtOC, isopropylsulfonyl, (2-N-Boc-amino)-ethoxycarbonyl, (5-carboxy)pentanoyl or (morpholin-4-yl)carboxyl; B is Phe, HPhe, Trp or Tyr (OMe); D is His, Ala, Ile, Leu, Met, Nle, Nva or Phe; G is Sta, ACHPA, HomoACHPA, AmACHPA or HomoAmACHPA; T is Arg, HArg, Orn, Dep, ($N^{\alpha}$Me)Lys or Lys; and J is 4-aminomethylpyridine, 4-(N-methyl)-aminomethylpyridine, 3-amino-2-oxoazepine or 4-aminomethylpiperidine.

4. A peptide according to Claim 3, wherein B is Phe, D is His, G is ACHPA or HomoACHPA and T is Lys, Orn, Dap or ($N^{\alpha}$-Me)Lys.

5. A pharmaceutical composition for renin associated hypertension or congestive heart failure comprising a pharmaceutical carrier and a therapeutically-effective amount of a peptide according to Claim 1.

6. A pharmaceutical composition according to Claim 4, also comprising an adjuvant.

7. A pharmaceutical composition according to Claim 4, also comprising one or more compounds selected from the group consisting of:

Diuretics:
acetazolamide; amiloride; bendroflumethiazide; benzthiazide; bumetanide; chlorothiazide; chlorthalidone; cyclothiazide; ethacrynic acid; furosemide; hydrochlorothiazide; hydroflumethiazide; indacrinone (racemic mixture, or as either the (+) or (-) enantiomer alone, or a manipulated ratio, e.g., 9:1 of said enantiomers, respectively); metolazone; methyclothiazide; muzolimine; polythiazide; quinethazone; sodium ethacrynate; sodium nitroprusside; spironolactone; ticrynafen; triamterene; trichlormethiazide;

α-Adrenergic Blocking Agents:
dibenamine; phentolamine; phenoxybenzamine; prazosin; tolazoline;

β-Adrenergic Blocking Agents:
atenolol; metoprolol; nadolol; propranolol; timolol;
((±)-2-[3-(tert-butylamino)-2-hydroxypropoxy]-2-furananilide) (ancarolol);
(2-acetyl-7-(2-hydroxy-3-isopropylaminopropoxy)benzofuran HCl) (befunolol);
((±)-1-(isopropylamino)-3-(p-(2-cyclopropylmethoxyethyl) phenoxy)-2-propranol HCl) (betaxolol);
(1-[(3,4-dimethoxyphenethyl)amino]-3-(m-tolyloxy)-2-propanol HCl) (bevantolol);
((±)-1-(4-((2-isopropoxyethoxy)methyl)phenoxy)-3-isopropylamino-2-propanol)fumarate) (bisoprolol);
(4-(2-hydroxy-3-[4-(phenoxymethyl)-piperidino]propoxy)-indole);
(carbazolyl-4-oxy-5,2-(2-methoxyphenoxy)-ethylamino-2-propanol);
(1-((1,1-dimethylethyl)amino)-3-((2-methyl-1H-indol-4-yl)oxy)-2-propanol benzoate) (bopindolol);
(1-(2-exobicyclo[2.2.1]-hept-2-ylphenoxy)-3-[(1-methylethyl)-amino]-2-propanol HCl) (bornaprolol);
(o-[2-hydroxy-3-[(2-indol-3-yl-1,1-dimethylethyl)amino]propoxy]benzonitrile HCl) (bucindolol);
(α-[(tert.butylamino)methyl]-7-ethyl-2-benzofuranmethanol) (bufuralol);
(3-[3-acetyl-4-[3-(tert.butylamino)-2-hydroxypropyl]phenyl]-1,1-diethylurea HCl) (celiprolol);
((±)-2-[2-[3-[(1,1-dimethylethyl)amino]-2-hydroxypropoxy]phenoxy]-N-methylacetamide HCl) (cetamolol);
(2-benzimidazolyl-phenyl(2-isopropylaminopropanol));
((±)-3′-acetyl-4′-(2-hydroxy-3-isopropylaminopropoxy)acetanilide HCl) (diacetolol);
(methyl-4-[2-hydroxy-3-[(1-methylethyl)aminopropoxy]]benzenepropanoate HCl) (esmolol);
(erythro-DL-1-(7-methylindan-4-yloxy)-3-isopropylaminobutan-2-ol);

(1-(tert.butylamino)-3-[O-(2-propynyloxy)phenoxy]-2-propanol (pargolol);
(1-(tert.butylamino)-3-[o-(6-hydrazino-3-pyridazinyl)phenoxy]-2-propanol diHCl) (prizidilol);
((-)-2-hydroxy-5-[(R)-1-hydroxy-2-[(R)-(1-methyl-3-phenylpropyl)amino]ethyl]benzamide);
(4-hydroxy-9-[2-hydroxy-3-(isopropylamino)-propoxy]-7-methyl-5H-furo[3,2-g][1]-benzopyran-5-one) (iprocrolol);
((-)-5-(tert.butylamino)-2-hydroxypropoxy]-3,4-dihydro-1-(2H)-naphthalenone HCl) (levobunolol);
(4-(2-hydroxy-3-isopropylamino-propoxy)-1,2-benzisothiazole HCl);
(4-[3-(tert.butylamino)-2-hydroxypropoxy]-N-methylisocarbostyril HCl);
((±)-N-2-[4-(2-hydroxy-3-isopropyl aminopropoxy)phenyl]ethyl-N′-isopropylurea) (pafenolol);
(3-[[(2-trifluoroacetamido)ethyl]amino]-1-phenoxypropan-2-ol);
(N-(3-(o-chlorophenoxy)-2-hydroxypropyl)-N′-(4′-chloro-2,3-dihydro-3-oxo-5-pyridazinyl)ethylenediamine);
((±)-N-[3-acetyl-4-[2-hydroxy-3-[(1-methylethyl)amino]propoxy]phenyl]butanamide) (acebutolol);
((±)-4′-[3-(tert-butylamino)-2-hydroxypropoxy]spiro[cyclohexane-1,2′-indan]-1′-one) (spirendolol);
(7-[3-[[2-hydroxy-3-[(2-methylindol-4-yl)oxy]propyl]amino]butyl]thiophylline) (teoprolol);
((±)-1-tert.butylamino-3-(thiochroman-8-yloxy)-2-propanol) (tertatolol);
((±)-1-tert.butylamino-3-(2,3-xylyloxy)-2-propanol HCl) (xibenolol);
(8-[3-(tert.butylamino)-2-hydroxypropoxy]-5-methylcoumarin) (bucumolol);
(2-(3-(tert.butylamino)-2-hydroxy-propoxy)benzonitrile HCl) (bunitrolol);
((±)-2′-[3-(tert-butylamino)-2-hydroxypropoxy-5′-fluorobutyrophenone) (butofilolol);
(1-(carbazol-4-yloxy)-3-(isopropylamino)-2-propanol) (carazolol);
(5-(3-tert.butylamino-2-hydroxy)propoxy-3,4-dihydrocarbostyril HCl) (carteolol);
(1-(tert.butylamino)-3-(2,5-dichlorophenoxy)-2-propanol) (cloranolol);
(1-(inden-4(or 7)-yloxy)-3-(isopropylamino)-2-propanol HCl) (indenolol);
(1-isopropylamino-3-[(2-methylindol-4-yl)oxy]-2-propanol) (mepindolol);
(1-(4-acetoxy-2,3,5-trimethylphenoxy)-3-isopropylaminopropan-2-ol) (metipranolol);
(1-(isopropylamino)-3-(o-methoxyphenoxy)-3-[(1-methylethyl)amino]-2-propanol) (moprolol);
((1-tert.butylamino)-3-[(5,6,7,8-tetrahydro-cis-6,7-dihydroxy-1-naphthyl)oxy]-2-propanol) (nadolol);
((S)-1-(2-cyclopentylphenoxy)-3-[(1,1-dimethylethyl)amino]-2-propanol sulfate (2:1)) (penbutolol);
(4′-[1-hydroxy-2-(amino)ethyl]methanesulfonanilide) (sotalol);
(2-methyl-3-[4-(2-hydroxy-3-tert.butylaminopropoxy)phenyl]-7-methoxy-isoquinolin-1-(2H)-one);
(1-(4-(2-(4-fluorophenyloxy)ethoxy)phenoxy)-3-isopropylamino-2-propanol HCl);
((-)-p-[3-[(3,4-dimethoxyphenethyl)amino]-2-hydroxypropoxy]-$\beta$-methylcinnamonitrile) (pacrinolol);
((±)-2-(3′-tert.butylamino-2′-hydroxypropylthio)-4-(5′-carbamoyl-2′-thienyl)thiazole HCl) (arotinolol);
((±)-1-[p-[2-(cyclopropylmethoxy)ethoxy]phenoxy]-3-(isopropylamino)-2-propanol) (cicloprolol);
((±)-1-[(3-chloro-2-methylindol-4-yl)oxy]-3-[(2-phenoxyethyl)amino]-2-propanol) (indopanolol);
((±)-6-[[2-[[3-(p-butoxyphenoxy)-2-hydroxypropyl]amino]ethyl]amino]-1,3-dimethyluracil) (pirepolol);
(4-(cyclohexylamino)-1-(1-naphtholenyloxy)-2-butanol);
(1-phenyl-3-[2-[3-(2-cyanophenoxy)-2-hydroxypropyl]aminoethyl]hydantoin HCl);
(3,4-dihydro-8-(2-hydroxy-3-isopropylaminopropoxy)-3-nitroxy-2H-1-benzopyran) (nipradolol);

<u>$\alpha$- and $\beta$-Adrenergic Blocking Agents:</u>

((±)-1-tert-butylamino)-3-[o-[2-(3-methyl-5-isoxazolyl)vinyl]phenoxy]-2-propanol) (isoxaprolol);
(1-isopropylamino-3-(4-(2-nitroxyethoxy)phenoxy)-2-propanol HCl);
(4-hydroxy-$\alpha$-[[3-(4-methoxyphenyl)-1-methylpropyl]aminomethyl]-3-(methylsulfinyl)-benzmethanol HCl) (sulfinalol);
(5-[1-hydroxy-2-[[2-(o-methoxyphenoxy)ethyl]amino]ethyl]-2-methylbenzenesulfonamide HCl);
(5-[1-hydroxy-2-[(1-methyl-3-phenylpropyl)amino]ethyl]salicylamide HCl) (labetalol);
(1-((3-chloro-2-methyl-1H-indol-4-yl)oxy)-3-((2-phenoxyethyl)amino)-2-propanol-hydrogenmalonate) (ifendolol);
(4-(2-hydroxy-3-[(1-methyl-3-phenylpropyl)amino]propoxy)benzeneacetamide);
(1-[3-[[3-(1-naphthoxy)-2-hydroxypropyl]-amino]-3,3-dimethyl-propyl]-2-benzimidazolinone);
(3-(1-(2-hydroxy-2-(4-chlorophenylethyl)-4-piperidyl)-3,4-dihydroxy)quinoxolin-2(1H)-one);

<u>CNS-Acting Agents:</u>

clonidine; methyldopa;

<u>Adrenergic Neuron Blocking Agents:</u>

guanethidine; reserpine and other rauwolfia alkaloids such as rescinnamine;

<u>Vasodilators:</u>

diazoxide; hydralazine; minoxidil;

<u>Angiotensin I Converting Enzyme Inhibitors:</u>

1-(3-mercapto-2-methyl-1-oxopropyl)-L-proline (captopril);
(1-(4-ethoxycarbonyl-2,4(R,R)-dimethylbutanoyl)indoline-2(S)-carboxylic acid);
(2-[2-[[1-(ethoxycarbonyl)-3-phenyl-propyl]amino]-1-oxopropyl]-1,2,3,4-tetrahydro-3- isoquinoline carboxylic acid);
((S)-1-[2-[[1-(ethoxycarbonyl)-3-phenylpropyl]amino]-1-oxopropyl]octahydro-1H-indole-2-carboxylic acid HCl);
(N-cyclopentyl-N-(3-(2,2-dimethyl-1-oxopropyl)thiol-2-methyl-1-oxopropyl)glycine) (pivalopril);
((2R,4R)-2-(2-hydroxyphenyl)-3-(3-mercaptopropionyl)-4-thiazolidinecarboxylic acid);
(1-(N-[1(S)-ethoxycarbonyl-3-phenylpropyl]-(S)-alanyl)cis,syn-octahydroindol-2(S)-carboxylic acid HCl);
((-)-(S)-1-[(S)-3-mercapto-2-methyl-1-oxopropyl]indoline-2-carboxylic acid);
([1(S),4S]-1-[3-(benzoylthio)-2-methyl-1-oxopropyl]-4-phenylthio-L-proline;
(3-([1-ethoxycarbonyl-3-phenyl-(1S)-propyl]amino)-2,3,4,5-tetrahydro-2-oxo-1-(3S)-benzazepine-1-acetic acid HCl;
(N-(2-benzyl-3-mercaptopropanoyl)-S-ethyl-L-cysteine) and the S-methyl analogue;
(N-(1(S)-ethoxycarbonyl-3-phenylpropyl)-L-alanyl-L-proline maleate) (enalapril);
N-[1-(S)-carboxy-3-phenylpropyl]-L-alanyl-1-proline;
$N^2$-[1-(S)-carboxy-3-phenylpropyl]-L-lysyl-L-proline (lisinopril);

Calcium Channel Blockers:

α-[3-[[2-(3,4-dimethoxyphenyl)ethyl]methylamino]propyl]-3,4-dimethoxy-α-(1-methylethyl)benzeneacetonitrile (verapamil);
1,4-dihydro-2,6-dimethyl-4-(2-nitrophenyl)-3,5-pyridinedicarboxylic acid dimethyl ester (nifedipine);
2-(2,2-dicyclohexylethyl)piperidine (perhexiline);
N-(1-methyl-2-phenylethyl)- -phenylbenzenepropanamine (prenylamine);
3-(aminosulfonyl)-4-chloro-N-(2,3-dihydro-2-methyl-1H-indol-1-yl)benzamide (indapamide);
(2′-(2-diethylaminoethoxy)-3-phenylpropiophenone (etafenone);
(4-[4,4-bis-(4-fluorophenyl)butyl]-N-(2,6-dimethylphenyl)-1-piperazineacetamide) (lidoflazine);
(2-(N-benzyl-N-methylamino)ethylmethyl-2,6-dimethyl-4-(m-nitrophenyl)-1,4-dihydro-3,5-pyridinedicarboxylate HCl) (nicardipine);
(N-(3,4-dimethoxyphenethyl)-2-(3,4-dimethoxyphenyl)-N-methyl-m-dithiane-2-propylamine-1,1,3,3-tetraoxide) (tiapamil);
(5,6-dimethoxy-2-(3-[(α-(3,4 dimethoxy)phenylethyl)methylamino]propyl)phthalimidine) (falipamil);
(β-[(2-methylpropoxy)methyl]-N-phenyl-N-phenylmethyl-1-pyrrolidineethanamine HCl monohydrate) (bepridil);
((+)-cis-3-(acetyloxy)-5-[2-(dimethylamino)ethyl]-2,3-dihydro-2-(4-methoxyphenyl)-1,5-benzothiazepin-4-(5H)-one)-(diltiazem);
((E)-1-[bis-(p-fluorophenyl)methyl]-4-cinnamylpiperazine di HCl) (flunarizine);
(5-[(3,4-dimethoxyphenethyl)methylamino]-2-isopropyl-2-(3,4,5-trimethoxyphenyl)valeronitrile (gallopamil);
(ethylmethyl(2,3-dichlorophenyl)-1,4-dihydro-2,6-dimethyl-3,5-pyridinedicarboxylate (felodipine);
(isopropyl-2-methoxyethyl-1,4-dihydro-2,6-dimethyl-4-(3-nitrophenyl)-3,5-pyridinecarboxylate) (nimodipine);
(3-ethyl-5-methyl-1,4-dihydro-2,6-dimethyl-4-(3-nitrophenyl)-3,5-pyridine-dicarboxylate) (nitrendipine); and

Other Antihypertensive Agents:

aminophylline; cryptenamine acetates and tannates; deserpidine; meremethoxylline procaine; pargyline; trimethaphan camsylate; and the like, as well as admixtures and combinations thereof.

8. The use of a peptide according to Claim 1 for the preparation of a medicament useful for treating renin-associated hypertension or congestive heart failure in mammals.

9. The use as claimed in Claim 8, wherein mammals are human and the therapeutically-effective amount is from 0.02 to 10 grams per day.